# EUROPEAN PATENT APPLICATION

(11) **EP 4 494 560 A1**
(43) Date of publication of application: **22.01.2025**
(21) Application number: 24189698.4
(22) Date of filing: 19.07.2024
(51) Int. Cl.: A61B 5/349, A61B 5/361, A61N 1/39, A61H 31/00

(54) **REMOVING ARTIFACTS FROM PHYSIOLOGICAL PARAMETERS**

(30) Priority: 20.07.2023 US 202363528066 P
(71) Applicant: Stryker Corporation, Portage, MI 49002-9711 (US)
(72) Inventor: CHAPMAN, Fred W., Kalamazoo, Michigan, 49002 (US)
(74) Representative: V.O.

(57) **Abstract**

An example method includes administering a treatment to a subject during a time interval. The example method further includes receiving, from an external device, a first signal indicating a physiological parameter of the subject detected during the time interval; removing, from the physiological parameter, an artifact associated with the treatment; and in response to removing the artifact from the physiological parameter, outputting a second signal indicating the physiological parameter.

## Description

### CROSS-REFERENCE TO RELATED APPLICATION

This application claims the priority of U.S. Provisional App. No. 63/528,066, which is titled "Removing Artifacts from Physiological Parameters," was filed on July 20, 2023, and is incorporated by reference herein in its entirety.

### BACKGROUND

Various medical problems cause individuals to suddenly and unpredictably lose consciousness. For example, an individual experiencing cardiac arrest loses consciousness when the individual's heart fails to effectively pump oxygenated blood to the individual's brain. In some cases, cardiac arrest occurs due to the heart exhibiting an arrhythmia, such as ventricular fibrillation (VF). Prolonged hypoxia of the individual's brain and other vital organs can cause permanent bodily harm or even death.

Rescuers may be deployed to the scene of the individual experiencing cardiac arrest. To prevent hypoxic injury, rescuers may administer chest compressions to the individual. Mechanical chest compression devices can be used to administer the chest compressions. Rescuers may further treat an arrhythmia causing the cardiac arrest (e.g., perform defibrillation) by administering an electrical shock to the individual's heart.

### SUMMARY

According to an aspect is provided a mechanical chest compression device. The device comprises a compressor configured to apply chest compressions to a subject. The device comprises a processor configured to identify a physiological parameter of the subject; remove, from the physiological parameter, an artifact associated with the chest compressions; and in response to removing the artifact from the physiological parameter, output the physiological parameter.

Optionally, the compressor comprises a piston or a chest band.

Optionally, the mechanical chest compression device comprises a sensor configured to detect the physiological parameter.

Alternatively, or additionally, the mechanical chest compression device is configured to receive, from a monitoring device, a signal indicating the physiological parameter.

Optionally, the physiological parameter comprises an electrocardiogram (ECG), a capnograph, a blood pressure, an airway flow, a blood flow, or a plethysmograph.

Optionally, the processor is configured to remove the artifact associated with the chest compressions by identifying a frequency of the chest compressions; generating a comb filter comprising a first band overlapping the frequency and second bands overlapping harmonics of the frequency; and applying the comb filter to the physiological parameter.

Optionally, the mechanical chest compression device comprises a transceiver. Optionally, transceiver is configured to receive, from a monitoring device, a signal indicating the physiological parameter. Optionally, the processor is configured to output the physiological parameter by causing the transceiver to transmit a signal indicating the physiological parameter to an external device.

Optionally, the mechanical chest compression device comprises an output device. The processor can be configured to output the physiological parameter by causing the output device to output a signal indicating the physiological parameter. The signal can comprise a visual signal and/or an audible signal.

Optionally, the processor is configured to in response to removing the artifact from the physiological parameter, determining, by analyzing the physiological parameter, that blood is spontaneously circulating in the subject. Optionally, the processor is configured to in response to determining that the blood is spontaneously circulating in the subject, causing the compressor to cease the chest compressions.

Optionally, the physiological parameter is an electrocardiogram (ECG), and the processor is further configured to: in response to removing the artifact from the ECG, determine that the ECG is indicative of ventricular fibrillation (VF) or pulseless ventricular tachycardia (VT); and in response to determining that the ECG is indicative of VF or pulseless VT, output, to a defibrillator, an instruction to administer an electrical shock to a heart of the subject.

Optionally, the defibrillator is a first defibrillator, the electrical shock is a first electrical shock, and the processor is further configured to: in response to determining that the ECG is indicative of VF or pulseless VT, output, to a second defibrillator, an instruction to administer a second electrical shock to the heart of the subject, the second shock having a different vector than the first shock.

Optionally, a time period between a leading edge of the first shock and a leading edge of the second shock is in a range of 1 millisecond (ms) to 200 ms.

Optionally, the second shock overlaps the first shock in time.

Optionally, determining that the ECG is indicative of VF or pulseless VT comprises: determining that an amplitude of the ECG has decreased greater than a threshold amount over a time period.

Optionally, the processor is further configured to: determine that the first defibrillator has previously output greater than a threshold number of electrical shocks to the subject.

Optionally, the mechanical chest compression device comprises a cot configured to support the subject.

According to an aspect is provided a system, comprising: a monitor configured to:
generate an electrocardiogram (ECG) by detecting an electrical signal indicative of cardiac activity of a subject; and to transmit a first signal indicating the ECG of the subject; and a mechanical chest compression device as described hereinabove. The processor can then be configured to: identify a frequency at which the compressor applies the chest compressions to the subject; generate a comb filter comprising a first band overlapping the frequency and second bands overlapping harmonics of the frequency; remove a chest compression artifact from the ECG by applying the comb filter to the ECG; and in response to removing the chest compression artifact from the ECG, cause the transceiver to transmit a second signal indicating the ECG of the subject omitting the chest compression artifact.

According to an aspect is provided a system, comprising: a monitor configured to:
generate an electrocardiogram (ECG) by detecting an electrical signal indicative of cardiac activity of a subject; and to transmit a first signal indicating the ECG of the subject; and a mechanical chest compression device comprising: a compressor configured to apply chest compressions to the subject; a transceiver configured to receive the first signal indicating the ECG of the subject; and a processor. The processor can be configured to: identify a frequency at which the compressor applies the chest compressions to the subject; generate a comb filter comprising a first band overlapping the frequency and second bands overlapping harmonics of the frequency; remove a chest compression artifact from the ECG by applying the comb filter to the ECG; and in response to removing the chest compression artifact from the ECG, cause the transceiver to transmit a second signal indicating the ECG of the subject omitting the chest compression artifact.

Optionally, the monitor is configured to detect a physiological parameter of the subject and to transmit a third signal indicating the physiological parameter. The transceiver can be configured to receive the third signal indicating the physiological parameter. The processor can be configured to: in response to removing the chest compression artifact from the ECG, determine that the ECG comprises a QRS complex; determine that the physiological parameter is indicative of blood flowing through a blood vessel of the subject, and in response to determining that the ECG comprises a QRS complex and determining that the physiological parameter is indicative of blood flowing through the blood vessel of the subject, cause the compressor to cease the chest compressions.

Optionally, the monitor is configured to: receive the second signal indicating the ECG of the subject omitting the chest compression artifact; and display the ECG of the subject omitting the chest compression artifact.

According to an aspect, is provided a method, comprising: receiving, from an external device, a first signal indicating a physiological parameter of the subject detected during a time interval in which a treatment is administered to a subject; removing, from the physiological parameter, an artifact associated with the treatment; and in response to removing the artifact from the physiological parameter, outputting a second signal indicating the physiological parameter.

Optionally, the treatment comprises chest compressions, assisted ventilation, or pace pulses. Optionally, the treatment comprises chest compressions, assisted ventilation, or pace pulses administered at a frequency.

Optionally, the physiological parameter comprises an electrocardiogram (ECG), a capnograph, a blood pressure, an airway flow, a blood flow, or a plethysmograph.

Optionally, removing the artifact associated with the treatment comprises: generating a comb filter comprising a first band overlapping a frequency of administration of the treatment and second bands overlapping harmonics of the frequency; and applying the comb filter to the physiological parameter.

Optionally, the treatment is a first treatment, the method comprising: in response to removing the artifact from the physiological parameter, determining, by analyzing the physiological parameter, that the subject has a treatable condition; and in response to determining that the subject has the treatable condition, generating an instruction to administer a second treatment and/or continue the first treatment.

Optionally, the method comprises: in response to removing the artifact from the physiological parameter, determining, by analyzing the physiological parameter, that the subject has a pulse or is spontaneously breathing; and in response to determining that the subject has the pulse or is breathing, generating an instruction to discontinue the treatment.

Optionally, physiological parameter is a first physiological parameter, and determining that the subject has the pulse or is spontaneously breathing comprises analyzing a second physiological parameter of the subject.

According to an aspect is provided a system, comprising: a mobile device; a mechanical chest compression device comprising: a compressor configured to apply chest compressions to a subject; a transceiver configured to receive a first signal indicating an electrocardiogram (ECG) of the subject; and a processor configured to: identify a frequency at which the compressor applies the chest compressions to the subject; generate a comb filter comprising a first band overlapping the frequency at second bands overlapping harmonics of the frequency; remove a chest compression artifact from the ECG by applying the comb filter to the ECG; and in response to removing the chest compression artifact from the ECG, cause the transceiver to transmit, to the mobile device, a second signal indicating the ECG of the subject omitting the chest compression artifact.

Optionally, the first signal further indicates the physiological parameter, and the processor is configured to: in response to removing the chest compression artifact from the ECG, determine that the ECG comprises a QRS complex; determine that the physiological parameter is indicative of blood flowing through a blood vessel of the subject, and in response to determining that the ECG comprises a QRS complex and determining that the physiological parameter is indicative of blood flowing through the blood vessel of the subject, cause the compressor to cease the chest compressions.

Optionally, the mobile device is configured to: receive the second signal indicating the ECG of the subject with the chest compression artifact removed; and display the ECG of the subject omitting the chest compression artifact.

According to an aspect is provided a monitor, comprising: a sensor configured to detect a physiological parameter comprising an artifact; a transceiver configured to transmit, to a treatment device, a first signal indicating the physiological parameter comprising the artifact and to receive, from the treatment device, a second signal indicating the physiological parameter without the artifact; and a display configured to visually present the physiological parameter without the artifact.

Optionally, the monitor comprises a monitor-defibrillator, and the treatment device comprises a mechanical chest compression device or a ventilation device.

Optionally, the physiological parameter comprises an electrocardiogram (ECG), a capnograph, or a plethysmograph.

Optionally, the artifact comprises a chest compression artifact, a ventilation artifact, or a motion artifact.

Optionally, the artifact is associated with a treatment administered by the treatment device.

Optionally, the monitor comprises: a processor configured to: determine, by analyzing the physiological parameter omitting the artifact, that a subject has a treatable condition; and in response to determining that the subject has the treatable condition, output a recommendation to administer a treatment to the subject.

Optionally, the treatable condition comprises ventricular fibrillation, bradycardia, or pulseless tachycardia.

Optionally, the monitor comprises: a treatment circuit configured to administer the treatment to the subject.

Optionally, the treatment comprises pace pulses or an electrical shock.

According to an aspect is provided a method, comprising: detecting a physiological parameter comprising an artifact; transmitting, to a treatment device, a first signal indicating the physiological parameter comprising the artifact; receiving, from the treatment device, a second signal indicating the physiological parameter omitting the artifact; and outputting, to a user, the physiological parameter omitting the artifact.

Optionally, the physiological parameter comprises electrocardiogram (ECG), a capnograph, or a plethysmograph.

Optionally, the artifact comprises a chest compression artifact, a ventilation artifact, or a motion artifact.

Optionally, the artifact is associated with a treatment administered by the treatment device.

Optionally, the method comprises: determining, by analyzing the physiological parameter omitting the artifact, that a subject has a treatable condition; and outputting an instruction to administer a treatment to the subject.

Optionally, the method comprises: receiving an input signal; and in response to receiving the input signal, generate an instruction to administer the treatment to the subject.

Optionally, the treatment comprises pace pulses, an electrical shock, assisted ventilation, or chest compressions.

Optionally, the method comprises: determining, by analyzing the physiological parameter without the artifact, that a subject has a pulse or is breathing spontaneously; and in response to determining that the subject has the pulse or is breathing spontaneously, transmitting, to the treatment device, an instruction to discontinue a treatment to the subject.

It will be appreciated that any of the aspects, features and options described herein can be combined. It will particularly be appreciated that any of the aspects, features and options described in view of the mechanical chest compression device apply equally to the systems, monitor and methods, and vice versa.

### BRIEF DESCRIPTION OF THE DRAWINGS

FIG. 1 illustrates a rescue scene utilizing artifact removal of a physiological parameter to enhance emergency care.
FIG. 2 illustrates an example comparison of an artifacted ECG and a filtered ECG.
FIG. 3 illustrates an example of a comb filter for removing a periodic artifact from a physiological parameter.
FIG. 4 illustrates an environment for administering a double-sequential defibrillation (DSD) therapy coordinated by a mechanical chest compression device.
FIG. 5 illustrates an example process for removing a treatment-induced artifact from a physiological parameter at the device performing the treatment.
FIG. 6 illustrates an example process for modifying a treatment by analyzing a physiological parameter.
FIG. 7 illustrates an example of an external defibrillator configured to perform various functions described herein.
FIG. 8 illustrates a chest compression device configured to perform various functions described herein.

### DETAILED DESCRIPTION

When caring for an individual in cardiac arrest, a rescuer may prioritize preventing hypoxic injury by administering chest compressions to the individual. However, it may be difficult to assess other conditions of a patient while the chest compressions are being administered. This may delay, or even prevent, the rescuer from identifying or treating the cause of the cardiac arrest. For example, a defibrillator or rescuer may analyze an electrocardiogram (ECG) of the individual to determine if the individual has a shockable arrhythmia (e.g., VF) that can be resolved by the administration of an electrical shock to the individual's heart. However, chest compressions introduce a motion artifact into the ECG that can make it difficult to analyze the ECG. Thus, the rescuer faced with a tradeoff between preventing hypoxic injury while the individual's heart is not pumping blood effectively (by administering chest compressions) and being able to diagnose and treat the underlying cause of the cardiac arrest (by identifying the shockable rhythm in the individual's ECG). It would be advantageous if the individual's ECG could be effectively analyzed while the individual is receiving chest compressions.

In some cases, artifacts such as chest compression artifacts can be removed from physiological parameters like ECG. Various signal processing techniques can be used to at least reduce artifacts. However, these techniques can have high computational costs, particularly if they are performed in real-time as the ECG is being detected. Various monitoring devices, such as legacy monitoring devices or inexpensive automated external defibrillators (AEDs), lack the processing capabilities to perform on-board chest compression artifact removal.

Various implementations described herein relate to techniques for a monitoring device that exports data indicating an artifacted physiological parameter and techniques for removing the artifact from the data based on a treatment parameter of a treatment device that is administering the treatment responsible for the artifact. The artifact can be removed, for example, by the treatment device itself or by another computing device that is separate from the monitoring device. Accordingly, the physiological parameter can be effectively analyzed simultaneously with the administration of the treatment.

Implementations of the present disclosure will now be described with reference to the accompanying figures.

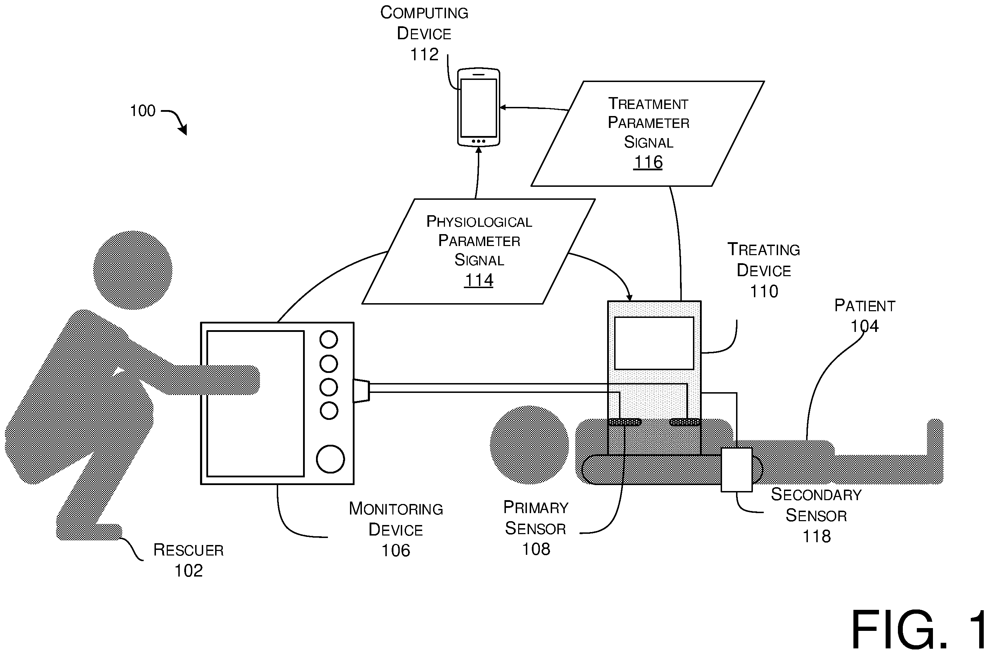

FIG. 1 illustrates a rescue scene 100 utilizing artifact removal of a physiological parameter to enhance emergency care. In various implementations, a rescuer 102 is treating a patient 104 at the rescue scene 100. For instance, the rescuer 102 is an emergency medical technician (EMT) professional monitoring and/or treating a medical condition of the patient 104. In some cases, the patient 104 has lost consciousness. For instance, the patient 104 is experiencing cardiac arrest or some other dangerous medical condition. The rescue scene 100, in various cases, is outside of a formal clinical environment. For instance, the rescue scene 100 may be in a non-clinical space, such as a school, an airport terminal, an outdoor space, or the like.

The rescuer 102 is monitoring the condition of the patient 104 using a monitoring device 106. For instance, the monitoring device 106 may be a monitor-defibrillator, an AED, a medical imaging device, an ultrasound monitor, a standalone ECG monitor (e.g., without defibrillation capabilities), or another type of patient monitor. The monitoring device 106 includes and/or is communicatively coupled to a primary sensor 108. The primary sensor 108 is configured to detect at least one physiological parameter of the patient 104. Examples of physiological parameters include, for instance, an ECG, an electrical impedance, a force administered to the patient 104, an acceleration of at least a portion of the patient 104, a blood pressure (e.g., invasive blood pressure, non-invasive blood pressure), an airway parameter (e.g., a partial pressure of carbon dioxide, a partial pressure of oxygen, a capnograph, an end tidal gas parameter, a flow velocity or rate of air in the airway of the patient 104 (also referred to as an "airway flow"), etc.), a blood oxygenation (e.g., a pulse oximetry value, a regional oximetry value, etc.), an electroencephalogram (EEG), a temperature, a heart sound, a blood flow rate, a physiological geometry (e.g., a shape of a blood vessel, an inner ear shape, etc.), a heart rate, a pulse rate, a blood flow (e.g., a velocity of blood, a volumetric flow rate of blood, etc.), or the like. For example, the primary sensor 108 includes at least one of electrodes, a detection circuit, defibrillator pads, a force sensor, an accelerometer, a blood pressure cuff, an ultrasound-based blood pressure sensor, an invasive (e.g., intra-arterial) blood pressure sensor (e.g., including a cannula inserted into the patient 104), a gas sensor (e.g., a carbon dioxide and/or oxygen sensor), a flowmeter, a pulse oximetry sensor a regional oximetry sensor, a thermometer, a microphone, an ultrasound transducer, a medical imaging device (e.g., an ultrasound imaging device), or the like. In various cases, the monitoring device 106 outputs the physiological parameter(s) to the rescuer 102. For instance, the monitoring device 106 includes a display, a speaker, or haptic feedback device that conveys the physiological parameter(s) to the rescuer 102.

A treating device 110 (also referred to as a "treatment device") administers a treatment to the patient 104. For example, the treating device 110 is a monitor-defibrillator, an AED, mechanical chest compression device, a smart bag-valve mask, a ventilator, a heart-lung machine, an intravenous fluid (IV) pump, or the like. Examples of treatments include defibrillation, pacing, cardioversion, administration of chest compressions, administration of oxygen to the airway of the patient 104, movement of air in the airway of the patient, administration of fluids to the patient 104, extracorporeal membrane oxygenation (ECMO), administration of a medication to the patient 104, or the like. In some implementations, the monitoring device 106 is also configured to administer a treatment to the patient 104. Further, in some cases, the treating device 110 includes or is communicatively coupled to the primary sensor 108.

The environment 100 also includes a computing device 112. In some cases, the computing device 112 is associated with, owned by, assigned to, or otherwise utilized by the rescuer 102. As used herein, the term "computing device," and its equivalents, can refer to a device that includes one or more processors configured to perform operations. In various cases, a computing device further includes memory configured to store instructions that are executed by the processor(s). For example, the computing device 112 may be a mobile phone, a tablet computer, a laptop computer, a wearable device, an Internet of Things (IoT) device, or some other type of portable user equipment. In some implementations, the computing device 112 is a nonportable device. For instance, the computing device 112 may be a server computer located remotely from the environment 100.

In some cases, the monitoring device 106, the treating device 110, or both are also portable devices. A portable device, for instance, includes an on-board power source (e.g., a battery). Various portable devices described herein may also be configured to be transported by hand to different locations. For example, each of the monitoring device 106 and the treating device 110 may be configured to be transported in a hand-held bag or backpack by the rescuer 102.

In some cases, the monitoring and/or treatment of the patient 104 is optimized by communication between the monitoring device 106 and the treating device 110. In particular examples, the monitoring device 106 or the treating device 110 reports a detected physiological parameter to the other device. In some cases, the monitoring device 106 or the treating device 110 reports a treatment parameter to the other device. The receiving device may perform one or more actions based on the physiological parameter and/or the treatment parameter. Actions performed by the monitoring device 106 or the treating device 110 include initiating a measurement of a physiological parameter at a particular time or frequency, outputting a signal to the rescuer 102, outputting a signal to the patient 104, performing a treatment at a particular time or frequency, adjusting a treatment parameter of an ongoing treatment, or any combination thereof.

According to some implementations, the monitoring device 106 or the treating device 110 instructs the other device to perform one or more actions. The receiving device, in turn, performs the action(s) based on the instruction from the monitoring device 106 or the treating device 110. For instance, the treating device 110 instructs the monitoring device 106 to administer an electrical shock to the patient 104 at a predetermined time. The treating device 110, in some cases, may cease chest compressions at the predetermined time. By exchanging reports, instructions, or other data, the monitoring device 106 and the treating device 110 can coordinate monitoring and treatment of the patient 104.

To exchange data, the monitoring device 106 and/or the treating device 110 are configured to establish and/or communicate via a communication channel. As used herein, the term "communication channel," and its equivalents, may refer to a medium over which a first endpoint (e.g., a sender) transmits information to one or more second endpoints (e.g., receivers). Examples of communication channels include wired connections, such as Ethernet or fiber optic paths, as well as wireless connections, such as Institute of Electronics and Electrical Engineers (IEEE) (e.g., WI-FI, BLUETOOTH, etc.) or 3^{rd} Generation Partnership Program (3GPP) (e.g., Long Term Evolution (LTE), New Radio (NR), etc.) connections. As used herein, the term "endpoint," and its equivalents, may refer to an entity that is configured to transmit and/or receive data. Examples of endpoints include user equipment (UE) (e.g., mobile phones, tablet computers, etc.), computers, base stations, access points (APs), servers, compute nodes, medical devices, Internet of Things (IoT) devices, and the like.

In some implementations, the communication channel between the monitoring device 106 and the treating device 110 is established when the monitoring device 106 and the treating device 110 are paired. In particular cases, the monitoring device 106 and first treating device 110 refrain from sharing substantive data (e.g., physiological metrics, reports about the patient 104, instructions for treating the patient 104, etc.) until the monitoring device 106 and the treating device 110 are paired. As used herein, the term "paired," and its equivalents, may refer to a state of multiple devices that have a shared link key that enables each device to cryptographically authenticate data it receives from any other device among the multiple devices.

In particular cases, a first paired device encrypts data prior to transmitting the data to a second paired device, and the second paired device restores the original data by decrypting the encrypted data. As used herein, the term "encrypt," and its equivalents, refers to a process of translating data from one format (e.g., an unencoded format) into an encoded format. In various cases, the encoded format is referred to as "ciphertext." Unencoded data, which has not been encrypted, may be referred to as being in "plaintext." In various examples, an entity encrypts data using at least one encryption key. An encryption key is a parameter that defines the translation of data from the one format into the encoded format. As used herein, the term "decrypt," and its equivalents, refers to a process of translating data from an encoded format into another format (e.g., an unencoded format), such as a plaintext format. In various examples, an entity encrypts data using at least one decryption key. A decryption key is a parameter that defines the translation of data from the encoded format into the other format. A link key, for example, is an encryption and/or decryption key.

Various cryptographic techniques can be utilized in accordance with the features described in this disclosure. For example, data can be encrypted and decrypted via a symmetric key, wherein the encryption key and the decryption key are equivalent. In some cases, data can be encrypted and decrypted via asymmetric keys, wherein the encryption key and the decryption key are different. Cryptographic hash functions (CHFs) are examples of cryptographic techniques. Examples of cryptographic techniques include the Data Encryption Standard (DES), Advanced Encryption Standard (AES), Elliptic Curve Cryptography (ECC), Rivest-Shamir-Adleman (RSA), Secure Hash Algorithm (SHA)-1, SHA-2, SHA-3, BLAKE, BLAKE2, BLAKE3, WHIRLPOOL, MD2, MD4, MD5, MD6, Temporal Key Integrity Protocol (TKIP), Rivest cipher 4 (RC4), variably modified permutation composition (VMPC), blowfish, Twofish, Threefish, Tiny Encryption Algorithm (TEA), Extended TEA (XTEA), Corrected Block TEA (XXTEA), Diffie-Hellman exchange (DHE), elliptic curve DHE, supersingular isogeny Diffie-Hellman (SIDH) key exchange, and so on. Any suitable encryption or decryption technique can be used in accordance with implementations of this disclosure.

Various mechanisms can be utilized to pair the monitoring device 106 with the treating device 110. For example, automated pairing may involve the exchange of data and/or pairing requests/responses between the devices 106 and 110. As another example, the monitoring device 106 may receive an input signal from an operator (e.g., the rescuer 102) that selects the treating device 110 as a device to pair with the monitoring device 106, or vice versa. In some cases, the monitoring device 106 detects an alternative signal (e.g., a flashing light pattern) from the treating device 110 that is indicated in a pairing request.

In some cases, the monitoring device 106 and the treating device 110 are paired, at least in part, based on signaling to and/or from the computing device 112. In a particular example, two or more devices can be brought into proximity to (e.g., into contact with) each other in order to facilitate pairing the monitoring device 106 with the treating device 110. For example, a "tap-to-pair" functionality may allow a user to bring the monitoring device 106 (or a component thereof) into close proximity to (e.g., into contact with) the treating device 110 (or a component thereof), or vice versa, and a short-range wireless protocol, such as BLUETOOTH, near-field communication (NFC), or the like, may be used to detect that the devices 106 and 110 are within a threshold distance of each other, and, in response, the devices 106 and 110 may be paired. In some cases, the computing device 112, such as a phone, may be brought into close proximity to (e.g., into contact with) the monitoring device 106 and/or the treating device 110 (e.g., by touching the intermediary device to both devices 106 and 110 sequentially), and a short-range wireless protocol may be used to detect these proximity events involving the computing device 112, and, in response, the devices 106 and 110 may be paired.

In various cases, the monitoring device 106 and the treating device 110 are paired when the treating device 110 transmits a pairing request to the monitoring device 106 and the monitoring device 106 pairs with the first treating device 110 based on the first pairing request. The pairing request, according to some cases, is part of a handshake between the monitoring device 106 and the treating device 110. As used herein, the term "handshake," and its equivalents, refers to one or more signals transmitted between at least two endpoints that establish protocols of communication between the endpoints (e.g., prior to substantive data being exchanged between the endpoints). The monitoring device 106 and the treating device 110, in some cases, utilize Transmission Control Protocol (TCP). TCP, for instance, utilizes handshakes to establish communication sessions between devices. For example, a first device transmits a synchronize message including a first sequence number to a second device. The second device, in turn, transmits a synchronize-acknowledgement message including a second sequence number as well as a first acknowledgement number that is one greater than the first sequence number. In response, the first device transmits an acknowledgement message including a second acknowledgment number that is one greater than the second sequence number to the second device. The first and second devices, in some examples, are paired once the first device transmits the acknowledgement message. The sequence and acknowledgement numbers are used to initialize counters that track messages and/or bytes of data transmitted between the paired devices.

In some examples, the pairing request includes or otherwise indicates a link key that enables the monitoring device 106 to pair with the treating device 110. The treating device 110, for instance, broadcasts the pairing request into the rescue scene 100. In some examples, the treating device 110 broadcasts the pairing request in response to receiving an input signal from the rescuer 102 (e.g., the rescuer has pushed a button on the treating device 110), in response to being powered on, in response to initiating the treatment of the patient 104, or the like.

According to some implementations, the monitoring device 106 outputs a physiological parameter signal 114 to the treating device 110 and to the computing device 112. The physiological parameter signal 114 illustrated in FIG. 1, in various cases, encodes data that indicates discrete measurements of physiological parameter(s) detected by the primary sensor 108. For example, the physiological parameter signal 114 indicates measurements of a physiological parameter of the patient 104 taken at a predetermined sampling rate. In some cases, the physiological parameter signal 114 represents a waveform. The monitoring device 106, for instance, encodes one or more data packets into the physiological parameter signal 114. In some implementations, the monitoring device 106 encodes a stream of data packets in the physiological parameter signal 114 that indicate updated measurements taken by the primary sensor 108.

In some examples, the treating device 110 may be administering a treatment to the patient 104 while the primary sensor 108 is detecting the physiological parameter and the monitoring device 106 is transmitting the physiological parameter signal 114. The treatment administered by the treating device 110, however, can generate an artifact in the detected physiological parameter, which is reflected in the physiological parameter signal 114. For instance, the treatment may cause the patient 104 to move while the primary sensor 108 is detecting the physiological parameter, which could generate a motion artifact reflected in the physiological parameter signal 114. For example, chest compressions or assisted ventilation performed by the treating device 110 may generate an artifact in an ECG detected by the primary sensor 108.

The artifact may prevent the monitoring device 106 from accurately identifying a condition of the patient 104. For example, if the monitoring device 106 is a defibrillator, the monitoring device 106 may be unable to determine whether the ECG of the patient 104 is indicative of a shockable arrhythmia, such as ventricular fibrillation (VF) or pulseless ventricular tachycardia (VT). Accordingly, the monitoring device 106 may be unable to output a recommendation to defibrillate the patient 104 in a timely fashion. Because shockable arrhythmias prevent adequate circulation of blood, the patient 104 may suffer hypoxemic injury due to the inability of the monitoring device 106 to accurately identify the condition in a timely fashion.

In some cases, if the monitoring device 106 outputs the physiological parameter signal 114 to the rescuer 102 (e.g., on a display), the artifact may prevent the rescuer from accurately assessing the condition of the patient 104. For instance, if the monitoring device 106 outputs a capnograph including a chest compression artifact to the rescuer 102, the artifact may cause rescuer 102 to misinterpret the capnograph as indicating that the patient 104 is spontaneously breathing, even though the patient 104 is not spontaneously breathing. In other examples, if the monitoring device 106 outputs a physiological parameter (e.g., blood pressure, blood flow, etc.) indicative of blood circulation to the rescuer 102 with a chest compression artifact, the artifact may cause the rescuer 102 to misinterpret the physiological parameter. For instance, the chest compression artifact may cause the rescuer 102 to presume that the patient 104 lacks spontaneous circulation when the patient 104 does, in fact, have a return of spontaneous circulation (ROSC). In some examples, the chest compression artifact may cause the rescuer 102 to presume that the patient 104 has spontaneous circulation, even though the heart of the patient 104 is not effectively circulating blood through the body of the patient 104.

In various implementations of the present disclosure, the treating device 110 is configured to remove a treatment-induced artifact from the physiological parameter signal 114 detected by the primary sensor 108. The treating device 110 may identify a treatment parameter regarding the treatment that the treating device 110, itself, is administering to the patient 104. The treating device 110 may further remove the treatment-induced artifact from data indicative of the physiological parameter detected by the primary sensor 108 based on the treatment parameter. As used herein, the term "treatment parameter," and its equivalents, may be a parameter indicating a magnitude, a frequency, a timing, a phase, a waveform, or other characteristic of a treatment. In some examples in which the treatment administered by the treating device 110 includes chest compressions, the treatment parameter includes a timing of the chest compressions, a frequency of the chest compressions, a type (e.g., the manufacturer and/or model) of the treating device 110 administering the chest compressions, or any combination thereof. In some instances in which the treatment administered by the treating device 110 includes assisted ventilation, the treatment parameter includes a waveform of the flow or pressure of the ventilations, a timing of the ventilations, a frequency of the ventilations, or any combination thereof.

In some implementations, the computing device 112 removes the artifact from the physiological parameter signal 114. For example, the treating device 110 reports the treatment parameter by transmitting a treatment parameter signal 116 to the computing device 112. Further, the computing device 112 receives the physiological parameter signal 114 from the monitoring device 106 or the treating device 110. Thus, the computing device 112 may remove the treatment-induced artifact from the data indicated by the physiological parameter signal 114 based on the treatment parameter indicated in the treatment parameter signal 116.

The treating device 110 and/or computing device 112 removes the artifact, in various implementations, by applying a digital or analog filter to the data indicated in the physiological parameter signal 114. The filter is generated by the treating device 110 and/or the computing device 112 based on the treatment parameter (e.g., indicated in the treatment parameter signal 116). Examples of filters include Bessel, bilinear, Butterworth, Cauer, Chebyshev, comb, finite impulse response, high-pass, low-pass, infinite impulse response, Linkwitz-Riley, notch, and Savitzky-Golay filters. In some examples, the treating device 110 and/or computing device 112 removes further noise from the physiological parameter signal 114 by applying one or more additional filters. In some cases, the treating device 110 and/or computing device 112 removes the artifact by converting the physiological parameter signal 114 from the time domain to the frequency domain (e.g., by performing a fast Fourier transform (FFT)), convolving the frequency domain representation of the physiological parameter signal 114 with the impulse response of the filter, and converting the resultant data into the time domain by performing an inverse FFT.

In particular implementations, the treating device 110 and/or computing device 112 removes the artifact by applying a comb filter to the physiological parameter signal 114. Chest compressions administered by certain mechanical chest compression devices, for example, produce an artifact in a physiological parameter with high regularity. A chest compression artifact caused by a mechanical chest compression device may be substantially removed by a comb filter or multiple notch filters. In various cases, the filter includes a reject band that overlaps a frequency at which the chest compressions are administered to the patient 104. In addition, the filter includes one or more additional reject bands that overlap harmonics of the frequency at which the chest compressions are administered to the patient 104. The frequency, for instance, is indicated in the treatment parameter signal 116.

In some cases, the treating device 110 and/or computing device 112 outputs the physiological parameter signal 114 with the artifact removed. For example, the treating device 110 includes a display that visually presents a waveform representing the physiological parameter signal 114 over time. In some cases, the treating device 110 transmits a filtered version of the physiological parameter signal 114 to the monitoring device 106 or the computing device 112 for further display or processing.

In some examples, the treating device 110 and/or computing device 112 determines a condition of the patient 104 by analyzing the physiological parameter signal 114 with the artifact removed. In some implementations, the treating device 110 determines that the physiological parameter signal 114 is indicative of an arrhythmia, QRS complexes, a pulse, a return of spontaneous circulation (ROSC), spontaneous breathing, or some other condition of the patient 104. For instance, the treating device 110 determines that the patient 104 has VF by determining that a particular frequency component (associated with VF) of an ECG of the patient 104 has greater than a first threshold magnitude. The treating device 110, in some cases, may determine that the ECG indicates development of low-amplitude VF (also referred to as "fine VF") if the amplitude of the ECG is below a second threshold magnitude. In some implementations, the treating device 110 determines that the ECG is exhibiting QRS complexes by determining that the ECG matches a predetermined spectral signature associated with QRS complexes.

In various cases, the treating device 110 and/or computing device 112 determines the condition of the patient 104 further based on an additional physiological parameter detected from a secondary sensor 118. For instance, the secondary sensor 118 is configured to detect blood flow, a blood pressure, a blood oxygenation, or an acceleration of the patient 104. In various implementations, the treating device and/or computing device 112 removes an artifact associated with the treatment administered by the treating device 110 from the additional physiological parameter. In some cases, the secondary sensor 118 is adhered to the skin of the patient 104. In some cases, the secondary skin 118 is strapped to or wrapped around a limb of the patient 104. For example, the limb is a finger, a wrist, an arm, an ankle, or a leg of the patient 104. In some implementations, the treating device 110 determines that blood is circulating in the patient 104 based on the additional physiological parameter.

In some cases, the treating device 110 determines that the patient 104 has a pulse based on the physiological parameter and the additional physiological parameter. For example, the treating device 110 may detect QRS complexes in the ECG of the patient 104 and determine that the blood pressure of the patient 104 is above a threshold, a blood oxygenation of the patient 104 is above a threshold, a flow velocity of blood in the patient 104 is above a threshold, or an acceleration of the patient 104 is above a threshold. That is, the treating device 110 may determine that the patient 104 has an organized heart rhythm and that the heart of the patient 104 is pumping blood effectively through the body of the patient 104.

In some examples, the treating device 110 determines that the patient 104 has pulseless electrical activity (PEA). For example, the treating device 110 may detect QRS complexes in the ECG of the patient 104, but may determine that the blood pressure of the patient 104 is below a threshold, a blood oxygenation of the patient 104 is below a threshold, a flow velocity of blood in the patient 104 is below a threshold, or an acceleration of the patient 104 is below a threshold. In some examples, the computing device 112 determines the condition of the patient 104 and reports the condition to the treating device 110.

According to various cases, the treating device 110 is configured to modify or pause the treatment based on the condition of the patient 104. The treating device 110 may generate instructions to modify or pause the treatment based on the condition of the patient 104. In some implementations, the treating device 110 changes a magnitude, frequency, position, duty cycle, or other parameter of the treatment based on the condition. For example, the treating device 110 may determine that the patient 104 is hypoxemic based on determining that a blood oxygenation of the patient 104 is below a threshold. In turn, the treating device 110 may increase the frequency of chest compressions administered to the patient 104, change the position of a compressor administering the chest compressions, increase a depth of the chest compressions, or the like. These changes may increase the circulation of oxygenated blood in the patient 104 and therefore prevent hypoxemic injury.

In some cases, the treating device 110 at least temporarily ceases the treatment based on the condition. The treating device 110 may generate instructions to at least temporarily cease the treatment based on the condition. For instance, the treating device 110 may determine that the patient 104 has a pulse based on detecting QRS complexes in the ECG of the patient 104 and also detecting pulsatile blood flow in a limb of the patient 104. Accordingly, the treating device 110 may pause administration of chest compressions to the patient 104.

In some examples, the treating device 110 and/or computing device 112 causes the monitoring device 106 to administer a treatment to the patient 104 based on the condition of the patient 104. For instance, although the monitoring device 106 may be incapable or otherwise unable to remove the artifact from the physiological parameter signal 114, but the treating device 110 may be able to do so. In some cases, the monitoring device 106 is a relatively simple or legacy device with minimal processing resources, and cannot perform complex filtering techniques. Thus, the treating device 110 may filter and analyze the physiological parameter signal 114 on behalf of the monitoring device 106. For instance, the monitoring device 106 may be a legacy AED that detects the ECG of the patient 104. The treating device 110 may be a mechanical chest compression device that administers chest compressions to the patient 104 and can remove a chest compression artifact form the ECG. In addition, the treating device 110 may determine that the ECG is indicative of VF. As a result, the treating device 110 may transmit a signal to the monitoring device 106 that causes the monitoring device 106 to administer an electrical shock to the patient 104. For instance, the treating device 110 may transmit, to the monitoring device 106, an instruction to administer an electrical shock or an ECG-like signal that would be interpreted as VF by the monitoring device 106.

Although FIG. 1 illustrates one monitoring device 106 and one treating device 110, implementations are not so limited. For example, the monitoring device 106 may be a first defibrillator, the treating device 110 may be a mechanical chest compression device, and a second defibrillator may also be configured to administer an electrical shock to the patient 104. For example, the first defibrillator is coupled to first defibrillation electrodes adhered to the chest of the patient 104 along a first vector, and the second defibrillator is coupled to second defibrillation electrodes adhered to the chest of the patient 104 along a second vector. In some cases, the treating device 110, upon removing an artifact from the ECG of the patient 104, may cause the administration of a double-sequential defibrillation (DSD) treatment. For example, the treating device 110 may determine that the ECG is indicative of VF and that the first defibrillator has administered greater than a threshold number of electrical shocks to the patient 104 without resolving the VF. In some cases, the treating device 110 may determine that the ECG has progressed from high-amplitude VF (i.e., "coarse" VF) to low-amplitude VF (i.e., "fine" VF). Based on one or both of these determinations, the treating device 110 may cause both the first defibrillator and the second defibrillator to administer electrical shocks to the patient 104 in a synchronized fashion. For example, the treating device 110 may cause the first defibrillator to administer a first electrical shock to the patient 104 and cause the second defibrillator to administer a second electrical shock to the patient 104, wherein the second electrical shock begins before the first electrical shock ends. For instance, the treating device 110 may transmit, to the first defibrillator and/or second defibrillator, instructions to that effect. In various implementations, a leading edge of the first electrical shock begins before a leading edge of the second electrical shock by a time interval in a range of 1 ms to 200 ms.

In some implementations, the computing device 112 is part of a cot configured to support the patient 104. As used herein, the term "cot," and its equivalents, may refer to an apparatus that includes a surface that supports a patient in a prone or head-elevated position. The surface, for instance, may be padded. The cot, for example, may be collapsible and configured to be stored in an ambulance or other patient transport apparatus. In some cases, the cot includes wheels that enable the cot and the patient 104 to be rolled from one location to another location. In various implementations, the computing device 112 in the cot facilitates communication between the computing device 112, the treating device 110, and the monitoring device 106 due to the physical proximity to the devices monitoring and/or treating the patient 104 at the same time.

According to some implementations, an entity including the monitoring device 106, the treating device 110, the computing device 112, or any combination thereof, identifies whether the patient 104 is moving based on techniques similar to those described elsewhere herein. For example, a motion sensor (e.g., an accelerometer, gyroscope, pressure sensor, or the like) is disposed on the body of the patient 104. In some cases, the motion sensor is part of a wearable device that is worn by the patient 104, part of the primary sensor 108, part of the secondary sensor 118, or any combination thereof. The motion sensor is configured to detect a movement (e.g., an acceleration) of the body of the patient 104. Like other physiological parameters, the movement detected by the motion sensor includes an artifact associated with a frequency-specific treatment administered to the patient 104, such as a chest compression artifact. In various implementations, the entity removes the artifact from data indicative of the motion detected by the motion sensor, in order to identify data indicative of non-treatment-induced motion of the patient 104. The non-treatment-induced motion, for instance, indicates whether the patient 104 is moving spontaneously (e.g., whether the patient is conscious), is experiencing a pathological movement (e.g., seizure), whether the patient 104 is being transported (e.g., whether the patient 104 is in a moving vehicle, such as an ambulance, or is being transported on the cot, etc.), or any combination thereof. For example, the entity may determine whether the patient 104 is moving by determining whether the non-treatment-induced motion of the patient 104 exceeds a threshold movement.

In some cases, the entity performs one or more additional actions based on a determination that the patient 104 is moving. In some cases, the entity is configured to remove an artifact from a physiological parameter of the patient 104 using a filtering mechanism. In some cases, the filtering mechanism is adaptive, such that non-treatment movement of the patient 104 could interfere with the efficacy of the filtering mechanism. In some examples, the entity disables the filtering mechanism in response to determining that the patient 104 is moving. In some cases, the entity ceases administration of the treatment, or some other treatment, in response to detecting that the patient 104 is moving. In some examples, the entity refrains from displaying the physiological parameter when it detects that the patient 104 is moving. Alternatively, if the entity determines that the patient 104 is substantially still (e.g., that the patient is not spontaneously moving, being transported, etc.), the entity may activate the filtering mechanism, cause or recommend administration of the treatment or some other treatment, display an indication of the physiological parameter, or any combination thereof.

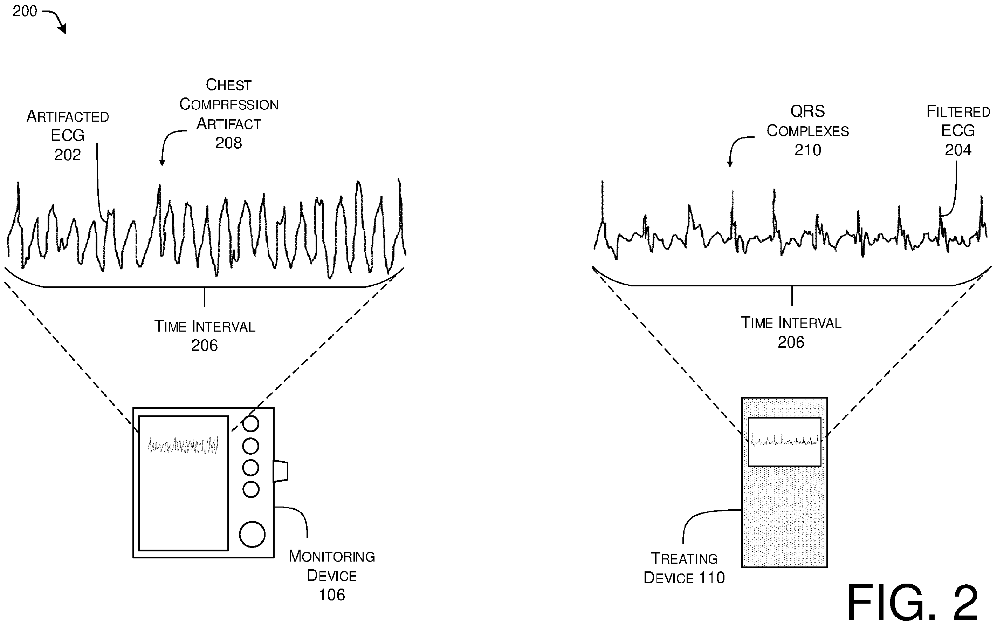

FIG. 2 illustrates an example comparison of an artifacted ECG 202 and a filtered ECG 204. As shown, FIG. 2 involves the monitoring device 106 and treating device 110 described above with reference to FIG. 1. In particular, the monitoring device 106 detects or otherwise identifies the artifacted ECG 202 of a patient (e.g., the patient 104). The artifacted ECG 202 is detected over a time interval 206. Notably, the artifacted ECG 202 includes a chest compression artifact 208 which obscures the underlying heart rhythm of the patient.

The monitoring device 106 transmits the artifacted ECG 202 to the treating device 110. The treating device 110, for instance, is administering the chest compressions that generate the chest compression artifact 208 in the artifacted ECG 202. The treating device 110 may remove the chest compression artifact 208 from the artifacted ECG 202, thereby generating the filtered ECG 204. In some examples, the treating device 110 generates a comb filter that includes a reject band overlapping the frequency of the chest compressions, as well as one or more additional reject bands respectively overlapping one or more harmonics of the frequency. The treating device 110 may remove the chest compression artifact 208 by applying the comb filter to the artifacted ECG 202.

The filtered ECG 204 can be output to a user and/or analyzed to determine a condition of the patient. In some cases, the treating device 110 outputs the filtered ECG 204 on a display. In some examples, the treating device 110 transmits the filtered ECG 204 to another device (e.g., the monitoring device 106 or some other computing device) that outputs the filtered ECG 204 on its display. According to various examples, the treating device 110 may detect the presence of QRS complexes 210 in the filtered ECG 204. In some cases, the QRS complexes 210 are evidence that the heart of the patient will spontaneously circulate blood, such that the tissues of the patient may be oxygenated without chest compressions. The treating device 110, for instance, may pause or otherwise cease the chest compressions in response to detecting the QRS complexes 210.

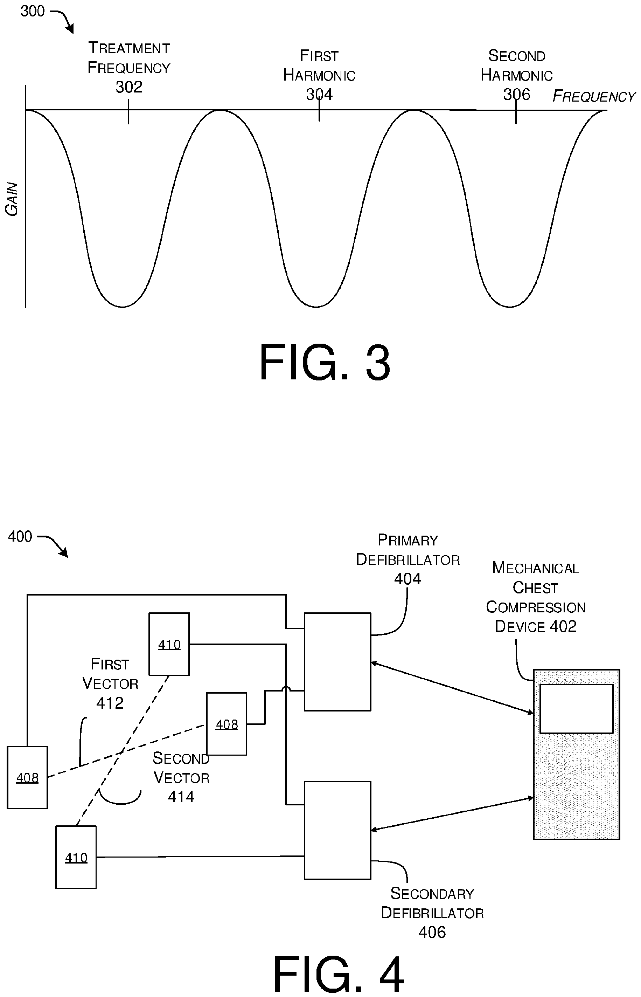

FIG. 3 illustrates an example of a comb filter 300 for removing a periodic artifact from a physiological parameter. For instance, the comb filter 300 may be used to remove an artifact caused by periodic assisted ventilation or chest compressions. The artifact may be caused by a treating device, such as a mechanical ventilation device or mechanical chest compression device.

The comb filter 300 may be represented in terms of frequency (x-axis) and gain (y-axis). As shown, the comb filter 300 includes a reject band, with negative gain, at a treatment frequency 302. The treatment frequency 302, for instance, is a frequency of assisted ventilation or chest compressions administered by the treating device. Further, the comb filter 300 includes additional reject bands, with negative gain, at a first harmonic 304 and a second harmonic 306 of the treatment frequency 302. Although the comb filter 300 is illustrated with only two reject bands at two harmonics of the treatment frequency 302, implementations of the present disclosure are not so limited. The comb filer 300, for instance, may include one or more reject bands overlapping one or more harmonics of the treatment frequency 302.

Due to the regularity of periodic treatments administered by devices, the comb filter 300 may be particularly suitable for removing artifacts associated with periodic treatments administered by devices. For instance, the comb filter 300 may be configured to substantially remove a chest compression artifact that is generated due to chest compressions administered by a mechanical chest compression device.

FIG. 4 illustrates an environment 400 for administering a DSD therapy coordinated by a mechanical chest compression device 402. The environment 400 further includes a primary defibrillator 402 and a secondary defibrillator 404. In various implementations, the mechanical chest compression device 402 corresponds to the treating device 110 described above with reference to FIG. 1, and the primary defibrillator 402 corresponds to the monitoring device 106 described above with reference to FIG. 1.

The primary defibrillator 402 is connected to first defibrillation electrodes 408. The secondary defibrillator 406 is connected to second defibrillation electrodes 410. In various cases, the first defibrillation electrodes 408 and the second defibrillation electrodes 410 are disposed on the chest of the same subject (e.g., a patient). A first vector 412 extends between the first defibrillation electrodes 408. A second vector 414 extends between the second defibrillation electrodes 410. In various implementations, the first vector 412 and the second vector 414 intersect the heart of the patient. In some cases, the first vector 412 and the second vector 414 intersect each other.

According to some cases, the mechanical chest compression device 402 may cause the primary defibrillator 404 to discharge a first electrical shock to the first defibrillation electrodes 408 and/or cause the secondary defibrillator 406 to output a second electrical shock to the second defibrillation electrodes 410. For example, the mechanical chest compression device 402 may transmit, to the primary defibrillator 404, a first signal that causes the primary defibrillator 404 to discharge the first electrical shock. In addition, the mechanical chest compression device 402 may transmit, to the secondary defibrillator 406, a second signal that causes the secondary defibrillator 406 to output the second electrical shock. The first signal and/or the second signal may be transmitted over one or more wired and/or wireless interfaces. In some cases, the first signal and the second signal include a synthesized ECG that includes a shockable arrhythmia (e.g., VF) that the primary defibrillator 404 and the secondary defibrillator 406 are configured to detect. For example, the primary defibrillator 404 and secondary defibrillator 406, upon detecting the shockable arrhythmia, are configured to output respective electrical shocks to the defibrillation electrodes. In some cases, the first signal and/or the second signal include an instruction to administer an electrical shock.

According to some implementations, the first electrical shock and the second electrical shock are output in a coordinated fashion. For instance, the mechanical chest compression device 402 may cause the secondary defibrillator 406 to output the second electrical shock before the first electrical shock has ceased. In other words, a leading edge of the second electrical shock occurs prior to a trailing edge of the first electrical shock.

In some cases, the mechanical chest compression device 404 causes the primary defibrillator 404 and the secondary defibrillator 406 to output respective electrical shocks in response to detecting an event. For instance, the mechanical chest compression device 402 may determine that an ECG of the patient (e.g., a filtered ECG of the patient) is indicative of VF. In addition, the mechanical chest compression device 402 may determine that the primary defibrillator 404 has already administered more than a threshold number (e.g., 3) electrical shocks in an attempt to defibrillate the patient. In some cases, the mechanical chest compression device 402 detects the electrical shocks in the ECG or by receiving a signal reporting the electrical shocks from the primary defibrillator 404 or secondary defibrillator 406. In some examples, the mechanical chest compression device 402 determines that the VF has transitioned from high-amplitude VF to low-amplitude VF. For example, the mechanical chest compression device 404 determines that an average amplitude of the VF rhythm during a first time period is above a threshold, but determines that an average amplitude of the VF rhythm during a second time period is below the threshold, wherein the second time period occurs after the first time period. In response to detecting the event(s), the mechanical chest compression device 402 may cause the primary defibrillator 404 and the second defibrillator 406 to output the first electrical shock and the second electrical shock as a DSD therapy.

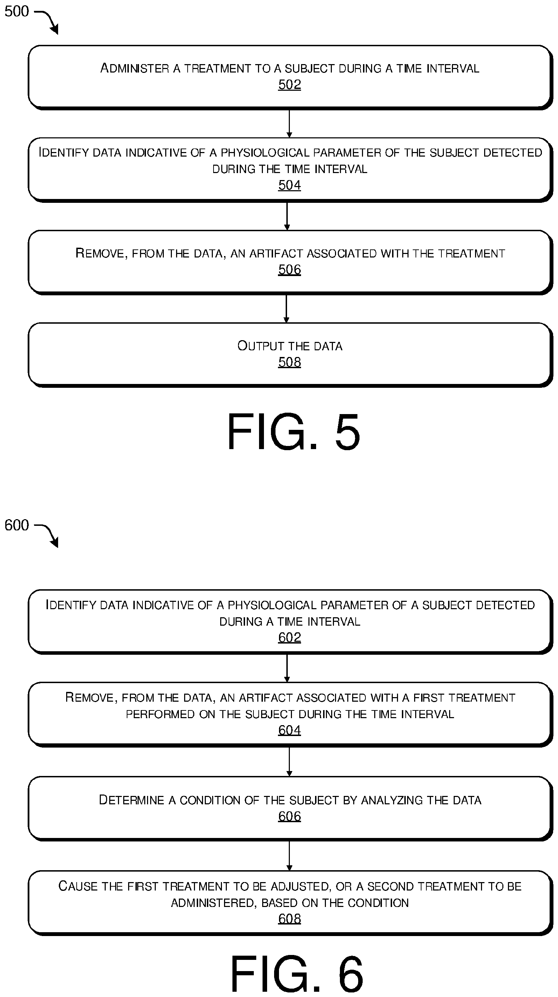

FIG. 5 illustrates an example process 500 for removing a treatment-induced artifact from a physiological parameter at the device performing the treatment. The process 500 may be performed by an entity including a processor, a medical device, a treating device, a mechanical chest compression device, a ventilator, or any combination thereof.

At 502 is indicated that the entity is administering a treatment to a subject during a time interval. The administering at 502 is not part of the process 500. Examples of the treatment include administration of an electrical shock (e.g., defibrillation), pacing, cardioversion, administration of chest compressions, administration of oxygen to the airway of the subject, movement of air in the airway of the patient, administration of fluids to the subject, ECMO, administration of a medication to the subject, or the like. According to various cases, the treatment causes a movement of the subject.

At 504, the entity identifies data indicative of a physiological parameter of the subject detected during the interval. In various cases, the entity receives a signal encoded with data indicating the physiological parameter from a separate device. For example, a monitoring device or sensor that is separate from the entity may detect the physiological parameter. Examples of the physiological parameter include an ECG, an electrical impedance, a force administered to the subject, an acceleration of at least a portion of the subject, a blood pressure, an airway parameter (e.g., a partial pressure of carbon dioxide, a partial pressure of oxygen, a capnograph, a plethysmograph, an end tidal gas parameter, a flow rate, etc.), a blood oxygenation (e.g., a pulse oximetry value, a regional oximetry value, etc.), an EEG, a temperature, a heart sound, a blood flow rate, a physiological geometry (e.g., a shape of a blood vessel, an inner ear shape, etc.), a heart rate, a blood flow (e.g., a blood velocity, volumetric flow rate, etc.) or a pulse rate. In some implementations, the entity itself detects the physiological parameter.

At 506, the entity removes, from the data, an artifact associated with the treatment. For example, the treatment-induced motion of the subject generates the artifact in the physiological parameter that is indicated in the data. In various cases, the entity identifies at least one treatment parameter characterizing the treatment. The treatment parameter, for instance, represents a magnitude, a position, a frequency, a timing, or a phase of the treatment. Based on the treatment parameter, the entity generates a filter configured to remove the artifact from the data. For instance, the entity generates a comb filter including a reject band overlapping a frequency of the treatment and one or more additional reject bands overlapping one or more harmonics of the frequency. In some cases, the entity convolves or cross-correlates the filter with the data in the frequency domain.

At 508, the entity outputs the data. In some cases, the entity visually presents the data on a display (e.g., a screen). For instance, the entity may display a waveform indicative of the data without the artifact. In various implementations, the entity transmits, to an external device, a signal indicative of the data. For instance, the entity may transmit the signal to the monitoring device or another computing device.

In some implementations, the entity, monitoring device, or other computing device may take one or more actions based on the data. In some cases, the entity may pause or alter the treatment based on an analysis of the data. The entity may transmit, to a treating device, a message that causes the treating device to pause or alter the treatment based on an analysis of the data. For instance, the entity may determine that the subject has a pulse by analyzing the data, and may cease administering chest compressions in response to detecting the pulse. In some implementations, the entity may transmit, to a treating device, a message that causes the treating device to administer another treatment based on an analysis of the data. For instance, the entity may determine that the subject has a shockable arrhythmia, and may cause an external device to administer an electrical shock to the subject's heart in response to detecting the shockable arrhythmia.

FIG. 6 illustrates an example process 600 for modifying a treatment by analyzing a physiological parameter. The process 600 may be performed by an entity including a processor, a computing device, a mobile device, a medical device, a treating device, a mechanical chest compression device, a ventilator, or any combination thereof.

At 602, the entity identifies data indicative of a physiological parameter of a subject detected during a time interval. In various cases, the entity receives a signal encoded with data indicating the physiological parameter from a separate device. For example, a monitoring device or sensor that is separate from the entity may detect the physiological parameter. Examples of the physiological parameter include an ECG, an electrical impedance, a force administered to the subject, an acceleration of at least a portion of the subject, a blood pressure, an airway parameter (e.g., a partial pressure of carbon dioxide, a partial pressure of oxygen, a capnograph, an end tidal gas parameter, a flow rate, a plythysmograph, etc.), a blood oxygenation (e.g., a pulse oximetry value, a regional oximetry value, etc.), an EEG, a temperature, a heart sound, a blood flow rate, a blood flow (e.g., a blood velocity, volumetric flow rate, etc.), a physiological geometry (e.g., a shape of a blood vessel, an inner ear shape, etc.), a heart rate, or a pulse rate. In some implementations, the entity itself detects the physiological parameter.

At 604, the entity removes, from the data, an artifact associated with a first treatment performed on the subject during the time interval. Examples of the first treatment include an electrical shock (e.g., defibrillation), pacing, cardioversion, administration of chest compressions, administration of oxygen to the airway of the subject, movement of air in the airway of the patient, administration of fluids to the subject, ECMO, administration of a medication to the subject, or the like. The first treatment, for instance, moves the subject such that a motion artifact is generated in the data indicative of the physiological parameter. In some cases, the entity administers the first treatment to the subject.

The entity may remove the artifact by generating a filter based on the first treatment. In various implementations, the entity identifies a treatment parameter that characterizes the first treatment. The treatment parameter, for instance, represents a magnitude, a position, a frequency, a timing, or a phase of the first treatment. Based on the treatment parameter, the entity generates a filter configured to remove the artifact from the data. For instance, the entity generates a comb filter including a reject band overlapping a frequency of the treatment and one or more additional reject bands overlapping one or more harmonics of the frequency. In some cases, the entity convolves or cross-correlates the filter with the data in the frequency domain.

At 606, the entity determines a condition of the subject by analyzing the data. With the artifact removed, the data may be indicative of the condition. In some cases, the entity determines whether the subject has a pulse. For example, the entity may determine that the subject has a pulse by determining that the ECG of the subject includes QRS complexes and/or a blood flow parameter (e.g., blood pressure, blood velocity, pulse wave velocity, blood oxygenation, etc.) is above a threshold. In some examples, the entity determines whether the subject has PEA. For example, the entity may determine that the subject has PEA by determining that the ECG of the subject includes QRS complexes, but that the blood flow parameter is below a threshold. In some cases, the entity may determine whether the subject is spontaneously breathing. For instance, the entity may determine that the subject is spontaneously breathing in response to determining that a capnograph of the subject includes periodic peaks indicative of spontaneous breathing. In some examples, the entity may determine whether the subject has VF by determining that a spectral signature of the ECG of the subject is consistent with VF. In various cases, the entity determines that the subject has pulseless VT by determining that the spectral signature of the ECG of the subject is consistent with VT and by determining that the blood flow parameter is below a threshold. In some case, the condition is whether the subject has low-amplitude or high-amplitude VF. For example, the entity may determine that the subject has low-amplitude VF by determining that an amplitude (e.g., a mean amplitude of one or more peaks) of the ECG of the subject is below a threshold.

At 608, the entity causes the first treatment to be adjusted, and/or a second treatment to be administered, based on the condition. The entity may transmit a message that causes the first treatment to be adjusted, and/or a second treatment to be administered, based on the condition. In some cases, the entity pauses or ends the first treatment based on the condition. In some examples, the entity modifies the treatment parameter based on the condition. According to various implementations, the entity may transmit, to an external device, a message causing the external device to administer the second treatment. Examples of the second treatment include an electrical shock (e.g., defibrillation), DSD, pacing, cardioversion, administration of chest compressions, administration of oxygen to the airway of the subject, movement of air in the airway of the patient, administration of fluids to the subject, ECMO, administration of a medication to the subject, or the like. In various implementations, the second treatment is a different type of treatment than the first treatment.

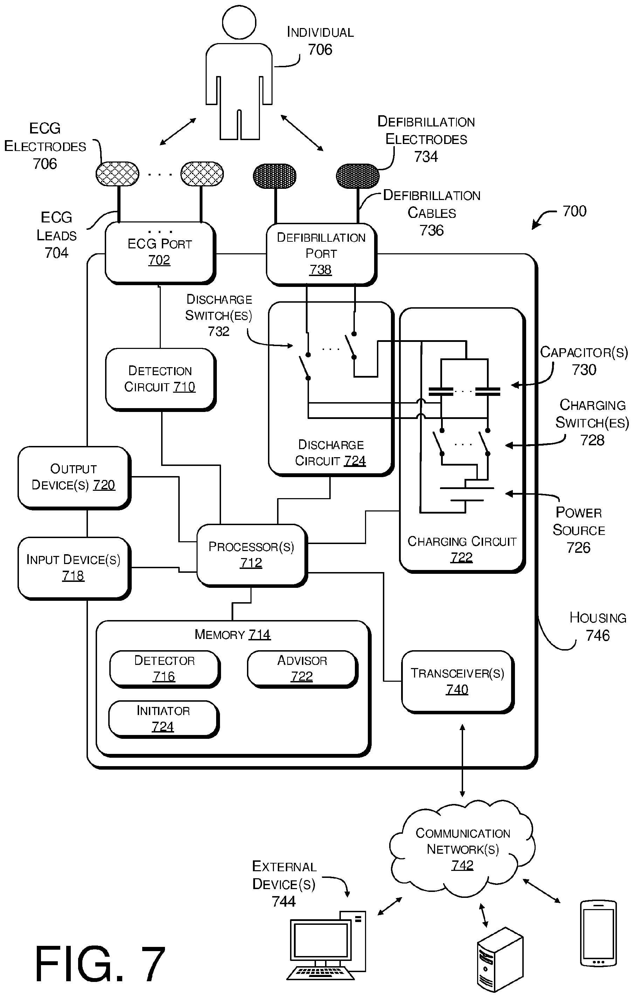

FIG. 7 illustrates an example of an external defibrillator 700 configured to perform various functions described herein. For example, the external defibrillator 700 is the monitoring device 106 described above with reference to FIG. 1.

The external defibrillator 700 includes an electrocardiogram (ECG) port 702 connected to multiple ECG leads 704. In some cases, the ECG leads 704 are removeable from the ECG port 702. For instance, the ECG leads 704 are plugged into the ECG port 702. The ECG leads 704 are connected to ECG electrodes 706, respectively. In various implementations, the ECG electrodes 706 are disposed on different locations on an individual 708. A detection circuit 710 is configured to detect relative voltages between the ECG electrodes 706. These voltages are indicative of the electrical activity of the heart of the individual 708.

In various implementations, the ECG electrodes 706 are in contact with the different locations on the skin of the individual 708. In some examples, a first one of the ECG electrodes 706 is placed on the skin between the heart and right arm of the individual 708, a second one of the ECG electrodes 706 is placed on the skin between the heart and left arm of the individual 708, and a third one of the ECG electrodes 706 is placed on the skin between the heart and a leg (either the left leg or the right leg) of the individual 708. In these examples, the detection circuit 708 is configured to measure the relative voltages between the first, second, and third ECG electrodes 706. Respective pairings of the ECG electrodes 706 are referred to as "leads," and the voltages between the pairs of ECG electrodes 706 are known as "lead voltages." In some examples, more than three ECG electrodes 706 are included, such that 5-lead or 12-lead ECG signals are detected by the detection circuit 710.

The detection circuit 710 includes at least one analog circuit, at least one digital circuit, or a combination thereof. The detection circuit 710 receives the analog electrical signals from the ECG electrodes 706, via the ECG port 702 and the ECG leads 704. In some cases, the detection circuit 710 includes one or more analog filters configured to filter noise and/or artifact from the electrical signals. The detection circuit 710 includes an analog-to-digital (ADC) in various examples. The detection circuit 710 generates a digital signal indicative of the analog electrical signals from the ECG electrodes 706. This digital signal can be referred to as an "ECG signal" or an "ECG."

In some cases, the detection circuit 710 further detects an electrical impedance between at least one pair of the ECG electrodes 706. For example, the detection circuit 710 includes, or otherwise controls, a power source that applies a known voltage (or current) across a pair of the ECG electrodes 706 and detects a resultant current (or voltage) between the pair of the ECG electrodes 706. The impedance is generated based on the applied signal (voltage or current) and the resultant signal (current or voltage). In various cases, the impedance corresponds to respiration of the individual 708, chest compressions performed on the individual 708, and other physiological states of the individual 708. In various examples, the detection circuit 710 includes one or more analog filters configured to filter noise and/or artifact from the resultant signal. The detection circuit 710 generates a digital signal indicative of the impedance using an ADC. This digital signal can be referred to as an "impedance signal" or an "impedance."

The detection circuit 710 provides the ECG signal and/or the impedance signal one or more processors 712 in the external defibrillator 700. In some implementations, the processor(s) 712 includes a central processing unit (CPU), a graphics processing unit (GPU), both CPU and GPU, or other processing unit or component known in the art.

The processor(s) 712 is operably connected to memory 714. In various implementations, the memory 712 is volatile (such as random access memory (RAM)), non-volatile (such as read only memory (ROM), flash memory, etc.) or some combination of the two. The memory 714 stores instructions that, when executed by the processor(s) 712, causes the processor(s) 712 to perform various operations. In various examples, the memory 714 stores methods, threads, processes, applications, objects, modules, any other sort of executable instruction, or a combination thereof. In some cases, the memory 714 stores files, databases, or a combination thereof. In some examples, the memory 714 includes, but is not limited to, RAM, ROM, electrically erasable programmable read-only memory (EEPROM), flash memory, or any other memory technology. In some examples, the memory 714 includes one or more of CD-ROMs, digital versatile discs (DVDs), content-addressable memory (CAM), or other optical storage, magnetic cassettes, magnetic tape, magnetic disk storage or other magnetic storage devices, or any other medium which can be used to store the desired information and which can be accessed by the processor(s) 712 and/or the external defibrillator 700. In some cases, the memory 714 at least temporarily stores the ECG signal and/or the impedance signal.

In various examples, the memory 714 includes a detector 716, which causes the processor(s) 712 to determine, based on the ECG signal and/or the impedance signal, whether the individual 708 is exhibiting a particular heart rhythm. For instance, the processor(s) 712 determines whether the individual 708 is experiencing a shockable rhythm that is treatable by defibrillation. Examples of shockable rhythms include ventricular fibrillation (VF) and ventricular tachycardia (V-Tach). In some examples, the processor(s) 712 determines whether any of a variety of different rhythms (e.g., asystole, sinus rhythm, atrial fibrillation (AF), etc.) are present in the ECG signal.

The processor(s) 712 is operably connected to one or more input devices 718 and one or more output devices 720. Collectively, the input device(s) 718 and the output device(s) 720 function as an interface between a user and the defibrillator 700. The input device(s) 718 is configured to receive an input from a user and includes at least one of a keypad, a cursor control, a touch-sensitive display, a voice input device (e.g., a microphone), a haptic feedback device (e.g., a gyroscope), or any combination thereof. The output device(s) 720 includes at least one of a display, a speaker, a haptic output device, a printer, or any combination thereof. In various examples, the processor(s) 712 causes a display among the input device(s) 718 to visually output a waveform of the ECG signal and/or the impedance signal. In some implementations, the input device(s) 718 includes one or more touch sensors, the output device(s) 720 includes a display screen, and the touch sensor(s) are integrated with the display screen. Thus, in some cases, the external defibrillator 700 includes a touchscreen configured to receive user input signal(s) and visually output physiological parameters, such as the ECG signal and/or the impedance signal.

In some examples, the memory 714 includes an advisor 722, which, when executed by the processor(s) 712, causes the processor(s) 712 to generate advice and/or control the output device(s) 720 to output the advice to a user (e.g., a rescuer). In some examples, the processor(s) 712 provides, or causes the output device(s) 720 to provide, an instruction to perform CPR on the individual 708. In some cases, the processor(s) 712 evaluates, based on the ECG signal, the impedance signal, or other physiological parameters, CPR being performed on the individual 708 and causes the output device(s) 720 to provide feedback about the CPR in the instruction. According to some examples, the processor(s) 712, upon identifying that a shockable rhythm is present in the ECG signal, causes the output device(s) 720 to output an instruction and/or recommendation to administer a defibrillation shock to the individual 708.

The memory 714 also includes an initiator 724 which, when executed by the processor(s) 712, causes the processor(s) 712 to control other elements of the external defibrillator 700 in order to administer a defibrillation shock to the individual 708. In some examples, the processor(s) 712 executing the initiator 724 selectively causes the administration of the defibrillation shock based on determining that the individual 708 is exhibiting the shockable rhythm and/or based on an input from a user (received, e.g., by the input device(s) 718). In some cases, the processor(s) 712 causes the defibrillation shock to be output at a particular time, which is determined by the processor(s) 712 based on the ECG signal and/or the impedance signal.

The processor(s) 712 is operably connected to a charging circuit 722 and a discharge circuit 724. In various implementations, the charging circuit 722 includes a power source 726, one or more charging switches 728, and one or more capacitors 730. The power source 726 includes, for instance, a battery. The processor(s) 712 initiates a defibrillation shock by causing the power source 726 to charge at least one capacitor among the capacitor(s) 730. For example, the processor(s) 712 activates at least one of the charging switch(es) 728 in the charging circuit 722 to complete a first circuit connecting the power source 726 and the capacitor to be charged. Then, the processor(s) 712 causes the discharge circuit 724 to discharge energy stored in the charged capacitor across a pair of defibrillation electrodes 730, which are in contact with the individual 708. For example, the processor(s) 712 deactivates the charging switch(es) 728 completing the first circuit between the capacitor(s) 730 and the power source 726, and activates one or more discharge switches 732 completing a second circuit connecting the charged capacitor 730 and at least a portion of the individual 708 disposed between defibrillation electrodes 734.

The energy is discharged from the defibrillation electrodes 734 in the form of a defibrillation shock. For example, the defibrillation electrodes 734 are connected to the skin of the individual 708 and located at positions on different sides of the heart of the individual 708, such that the defibrillation shock is applied across the heart of the individual 708. The defibrillation shock, in various examples, depolarizes a significant number of heart cells in a short amount of time. The defibrillation shock, for example, interrupts the propagation of the shockable rhythm (e.g., VF o pulseless VT) through the heart. In some examples, the defibrillation shock is 200 J or greater with a duration of about 0.015 seconds. In some cases, the defibrillation shock has a multiphasic (e.g., biphasic) waveform. The discharge switch(es) 732 are controlled by the processor(s) 712, for example. In various implementations, the defibrillation electrodes 734 are connected to defibrillation cables 736. The defibrillation cables 736 are connected to a defibrillation port 738, in implementations. According to various examples, the defibrillation cables 736 are removable from the defibrillation port 738. For example, the defibrillation cables 736 are plugged into the defibrillation port 738.

In various implementations, the processor(s) 712 is operably connected to one or more transceivers 740 that transmit and/or receive data over one or more communication networks 742. For example, the transceiver(s) 740 includes a network interface card (NIC), a network adapter, a local area network (LAN) adapter, or a physical, virtual, or logical address to connect to the various external devices and/or systems. In various examples, the transceiver(s) 740 includes any sort of wireless transceivers capable of engaging in wireless communication (e.g., radio frequency (RF) communication). For example, the communication network(s) 742 includes one or more wireless networks that include a 3^{rd} Generation Partnership Project (3GPP) network, such as a Long Term Evolution (LTE) radio access network (RAN) (e.g., over one or more LTE bands), a New Radio (NR) RAN (e.g., over one or more NR bands), or a combination thereof. In some cases, the transceiver(s) 740 includes other wireless modems, such as a modem for engaging in WI-FI^{®}, WIGIG^{®}, WIMAX^{®}, BLUETOOTH^{®}, or infrared communication over the communication network(s) 742.

The defibrillator 700 is configured to transmit and/or receive data (e.g., ECG data, impedance data, data indicative of one or more detected heart rhythms of the individual 708, data indicative of one or more defibrillation shocks administered to the individual 708, data indicative of one or more physiological parameters of the individual 708, etc.) with one or more external devices 744 via the communication network(s) 742. The external devices 744 include, for instance, mobile devices (e.g., mobile phones, smart watches, etc.), loT devices, medical devices (e.g., mechanical chest compression devices), computers (e.g., laptop devices, servers, etc.), or any other type of computing device configured to communicate over the communication network(s) 742. In some examples, the external device(s) 744 is located remotely from the defibrillator 700, such as at a remote clinical environment (e.g., a hospital). According to various implementations, the processor(s) 712 causes the transceiver(s) 740 to transmit data to the external device(s) 744. In some cases, the transceiver(s) 740 receives data from the external device(s) 744 and the transceiver(s) 740 provide the received data to the processor(s) 712 for further analysis.

In various implementations, the external defibrillator 700 also includes a housing 746 that at least partially encloses other elements of the external defibrillator 700. For example, the housing 746 encloses the detection circuit 710, the processor(s) 712, the memory 714, the charging circuit 722, the transceiver(s) 740, or any combination thereof. In some cases, the input device(s) 718 and output device(s) 720 extend from an interior space at least partially surrounded by the housing 746 through a wall of the housing 746. In various examples, the housing 746 acts as a barrier to moisture, electrical interference, and/or dust, thereby protecting various components in the external defibrillator 700 from damage.

In some implementations, the external defibrillator 700 is an automated external defibrillator (AED) operated by an untrained user (e.g., a bystander, layperson, etc.) and can be operated in an automatic mode. In automatic mode, the processor(s) 712 automatically identifies a rhythm in the ECG signal, makes a decision whether to administer a defibrillation shock, charges the capacitor(s) 730, discharges the capacitor(s) 730, or any combination thereof. In some cases, the processor(s) 712 controls the output device(s) 720 to output (e.g., display) a simplified user interface to the untrained user. For example, the processor(s) 712 refrains from causing the output device(s) 720 to display a waveform of the ECG signal and/or the impedance signal to the untrained user, in order to simplify operation of the external defibrillator 700.

In some examples, the external defibrillator 700 is a monitor-defibrillator utilized by a trained user (e.g., a clinician, an emergency responder, etc.) and can be operated in a manual mode or the automatic mode. When the external defibrillator 700 operates in manual mode, the processor(s) 712 cause the output device(s) 720 to display a variety of information that may be relevant to the trained user, such as waveforms indicating the ECG data and/or impedance data, notifications about detected heart rhythms, and the like.

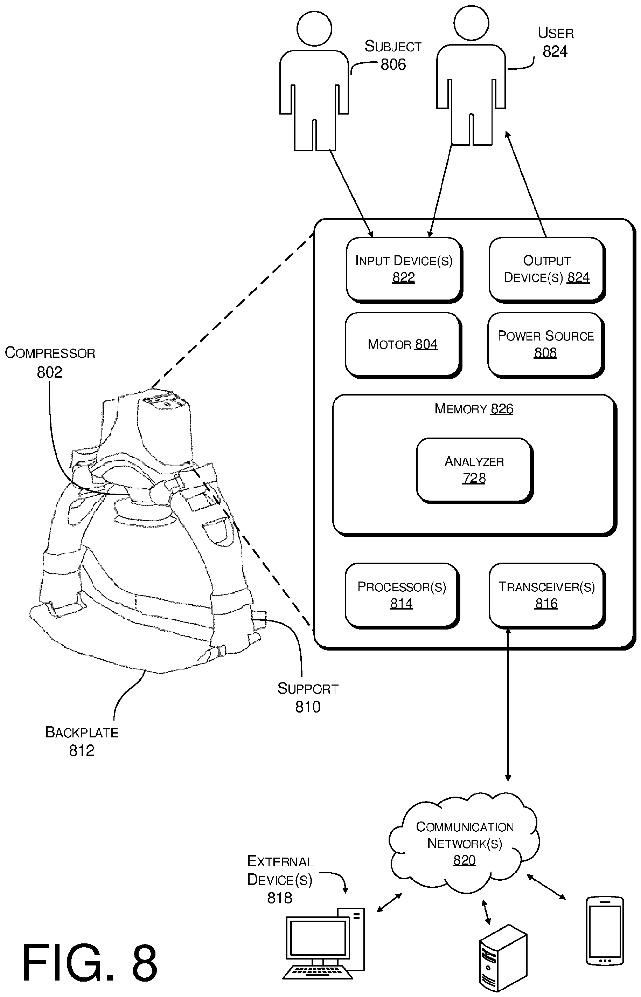

FIG. 8 illustrates a chest compression device 800 configured to perform various functions described herein. For example, the chest compression device 800 is the treating device 110 described with reference to FIG. 1.

In various implementations, the chest compression device 800 includes a compressor 802 that is operatively coupled to a motor 804. The compressor 802 is configured to physically administer a force to the chest of a subject 806 that compresses the chest of the subject 806. In some examples, the compressor 802 includes at least one piston that periodically moves between two positions (e.g., a compressed position and a release position) at a compression frequency. For example, when the piston is positioned on the chest of the subject 806, the piston compresses the chest when the piston is moved into the compressed position. A suction cup may be positioned on a tip of the piston, such that the suction cup contacts the chest of the subject 806 during operation. In various cases, the compressor 802 includes a band that periodically tightens to a first tension and loosens to a second tension at a compression frequency. For instance, when the band is disposed around the chest of the subject 806, the band compresses the chest when the band tightens.

The motor 804 is configured to convert electrical energy stored in a power source 808 into mechanical energy that moves and/or tightens the compressor 802, thereby causing the compressor 802 to administer the force to the chest of the subject 806. In various implementations, the power source 808 is portable. For instance, the power source 808 includes at least one rechargeable (e.g., lithium-ion) battery. In some cases, the power source 808 supplies electrical energy to one or more elements of the chest compression device 800 described herein.

In various cases, the chest compression device 800 includes a support 810 that is physically coupled to the compressor 802, such that the compressor 802 maintains a position relative to the subject 806 during operation. In some implementations, the support 810 is physically coupled to a backplate 812, cot, or other external structure with a fixed position relative to the subject 806. According to some cases, the support 810 is physically coupled to a portion of the subject 806, such as wrists of the subject 806.

The operation of the chest compression device 800 may be controlled by at least one processor 814. In various implementations, the motor 806 is communicatively coupled to the processor(s) 814. Specifically, the processor(s) 814 is configured to output a control signal to the motor 806 that causes the motor 806 to actuate the compressor 802. For instance, the motor 806 causes the compressor 802 to administer the compressions to the subject 806 based on the control signal. In some cases, the control signal indicates one or more treatment parameters of the compressions. Examples of treatment parameters include a frequency, timing, depth, force, position, velocity, and acceleration of the compressor 802 administering the compressions. According to various cases, the control signal causes the motor 806 to cease compressions.

In various implementations, the chest compression device 800 includes at least one transceiver 816 configured to communicate with at least one external device 818 over one or more communication networks 820. Any communication network described herein can be included in the communication network(s) 820 illustrated in FIG. 8. The external device(s) 818, for example, includes at least one of a monitoring device, a monitor-defibrillator, an AED, an ECMO device, a ventilation device, a patient monitor, a mobile phone, a server, or a computing device. In some implementations, the transceiver(s) 816 is configured to communicate with the external device(s) 818 by transmitting and/or receiving signals wirelessly. For example, the transceiver(s) 816 includes a NIC, a network adapter, a LAN adapter, or a physical, virtual, or logical address to connect to the various external devices and/or systems. In various examples, the transceiver(s) 816 includes any sort of wireless transceivers capable of engaging in wireless communication (e.g., RF communication). For example, the communication network(s) 820 includes one or more wireless networks that include a 3GPP network, such as an LTE RAN (e.g., over one or more LTE bands), an NR RAN (e.g., over one or more NR bands), or a combination thereof. In some cases, the transceiver(s) 816 includes other wireless modems, such as a modem for engaging in WI-FI^{®}, WIGIG^{®}, WIMAX^{®}, BLUETOOTH^{®}, or infrared communication over the communication network(s) 820. The signals, in various cases, encode data in the form of data packets, datagrams, or the like. In some cases, the signals are transmitted as compressions are being administered by the chest compression device 800 (e.g., for real-time feedback by the external device(s) 818), after compressions are administered by the chest compression device 800 (e.g., for post-event review at the external device 818), or a combination thereof. In various implementations, the transceiver(s) 816 are configured to receive signals encoded with one or more physiological parameters from the external device(s) 818.

In various cases, the processor(s) 814 generates the control signal based on data encoded in the signals received from the external device(s) 818. For instance, the signals include an instruction to initiate the compressions, and the processor(s) 814 instructs the motor 806 to begin actuating the compressor 802 in accordance with the signals.

In some cases, the chest compression device 800 includes at least one input device 822. In various examples, the input device(s) 822 is configured to receive an input signal from a user 824, who may be a rescuer treating the subject 806. Examples of the input device(s) 822 include, for instance, at a keypad, a cursor control, a touch-sensitive display, a voice input device (e.g., a microphone), a haptic feedback device (e.g., a gyroscope), or any combination thereof. In various implementations, the processor(s) 814 generate the control signal based on the input signal. For instance, the processor(s) 814 generate the control signal to adjust a frequency of the compressions based on the chest compression device 800 detecting a selection by the user 824 of a user interface element displayed on a touchscreen or detecting the user 824 pressing a button integrated with an external housing of the chest compression device 800.

According to some examples, the input device(s) 822 include one or more sensors. The sensor(s), for example, is configured to detect a physiological parameter of the subject 806. In some implementations, the sensor(s) is configured to detect a state parameter of the chest compression device 800, such as a position of the compressor 802 with respect to the subject 806 or the backplate 812, a force administered by the compressor 802 on the subject 806, a force administered onto the backplate 822 by the body of the subject 806 during a compression, or the like. According to some implementations, the signals transmitted by the transceiver(s) 816 indicate the physiological parameter(s) and/or the state parameter(s).

The chest compression device 800 further includes at least one output device 824, in various implementations. Examples of the output device(s) 824 include, for instance, least one of a display (e.g., a projector, an LED screen, etc.), a speaker, a haptic output device, a printer, or any combination thereof. In some implementations, the output device(s) 824 include a screen configured to display various parameters detected by and/or reported to the chest compression device 800, a charge level of the power source 808, a timer indicating a time since compressions were initiated or paused, and other relevant information.

The chest compression device 800 further includes memory 826. In various implementations, the memory 826 is volatile (such as RAM), non-volatile (such as ROM, flash memory, etc.) or some combination of the two. The memory 826 stores instructions that, when executed by the processor(s) 814, causes the processor(s) 814 to perform various operations. In various examples, the memory 826 stores methods, threads, processes, applications, objects, modules, any other sort of executable instruction, or a combination thereof. In some cases, the memory 826 stores files, databases, or a combination thereof. In some examples, the memory 826 includes, but is not limited to, RAM, ROM, EEPROM, flash memory, or any other memory technology. In some examples, the memory 826 includes one or more of CD-ROMs, DVDs, CAM, or other optical storage, magnetic cassettes, magnetic tape, magnetic disk storage or other magnetic storage devices, or any other medium which can be used to store the desired information. In various cases, the memory 826 stores instructions, programs, threads, objects, data, or any combination thereof, that cause the processor(s) 814 to perform various functions. In various cases, the memory 826 stores one or more parameters that are detected by the chest compression device 800 and/or reported to the chest compression device 800.

In implementations of the present disclosure, the memory 826 also stores instructions for executing various functionality described herein. For instance, the memory 826 includes an analyzer 728. When executed by the processor(s) 814, in various implementations, the analyzer 728 may cause the processor(s) 814 to identify a treatment parameter of chest compressions administered by the compressor 802. For example, the treatment parameter may be a compression depth, a timing, a frequency, a phase, or a position of the chest compressions. The analyzer 728 may further cause the processor(s) 814 to remove a chest compression artifact from data indicative of one or more physiological parameters (e.g., transmitted from the external device(s) 818 and/or detected by a sensor in the input device(s) 822). In some implementations, the analyzer 728 further determines a condition of a subject based on the filtered physiological parameter(s). Further, the analyzer 728 may cause the processor(s) 814 to identify a condition of a subject receiving the chest compressions based on the physiological parameter(s). In some cases, the analyzer 728 may cause the processor(s) 814 to pause the chest compressions administered by the compressor 802 based on the condition. In various implementations, the transceiver(s) 816 transmits data indicative of the treatment parameter, the physiological parameter(s), the condition, or a message that causes the external device(s) 818 to perform an action based on the condition.

### EXAMPLE CLAUSES

1. A system, including: a monitor configured to detect an electrocardiogram (ECG) of a subject and to transmit a first signal indicating the ECG of the subject; and a mechanical chest compression device including: a compressor configured to apply chest compressions to the subject; a transceiver configured to receive the first signal indicating the ECG of the subject; and a processor configured to: identify a frequency at which the compressor applies the chest compressions to the subject; generate a comb filter including a first band overlapping the frequency and second bands overlapping harmonics of the frequency; remove a chest compression artifact from the ECG by applying the comb filter to the ECG; and in response to removing the chest compression artifact from the ECG, cause the transceiver to transmit a second signal indicating the ECG of the subject omitting the chest compression artifact.
2. The system of clause 1, wherein the monitor is further configured to detect a physiological parameter of the subject and to transmit a third signal indicating the physiological parameter, wherein the transceiver is further configured to receive the third signal indicating the physiological parameter, and wherein the processor is further configured to: in response to removing the chest compression artifact from the ECG, determine that the ECG includes a QRS complex; determine that the physiological parameter is indicative of blood flowing through a blood vessel of the subject, and in response to determining that the ECG includes a QRS complex and determining that the physiological parameter is indicative of blood flowing through the blood vessel of the subject, cause the compressor to cease the chest compressions.
3. The system of clause 1 or 2, wherein the monitor is further configured to: receive the second signal indicating the ECG of the subject omitting the chest compression artifact; and display the ECG of the subject omitting the chest compression artifact.
4. A mechanical chest compression device, including: a compressor configured to apply chest compressions to a subject; and a processor configured to: identify a physiological parameter of the subject; remove, from the physiological parameter, an artifact associated with the chest compressions; and in response to removing the artifact from the physiological parameter, outputting the physiological parameter.
5. The mechanical chest compression device of clause 4, wherein the compressor includes a piston or a chest band.
6. The mechanical chest compression device of clause 4 or 5, further including: a sensor configured to detect the physiological parameter.
7. The mechanical chest compression device of one of clauses 4 to 6, further including: a transceiver configured to receive, from a monitoring device, a signal indicating the physiological parameter.
8. The mechanical chest compression device of one of clauses 4 to 7, wherein the physiological parameter includes an electrocardiogram (ECG), a capnograph, a blood pressure, an airway flow, a blood flow, or a plethysmograph.
9. The mechanical chest compression device of one of clauses 4 to 8, wherein the processor is configured to remove the artifact associated with the chest compressions by: identifying a frequency of the chest compressions; generating a comb filter including a first band overlapping the frequency and second bands overlapping harmonics of the frequency; and applying the comb filter to the physiological parameter.
10. The mechanical chest compression device of one of clauses 4 to 9, further including: a transceiver, wherein the processor is configured to output the physiological parameter by causing the transceiver to transmit a signal indicating the physiological parameter to an external device.
11. The mechanical chest compression device of one of clauses 4 to 10, further including: an output device, wherein the processor is configured to output the physiological parameter by causing the output device to output a signal indicating the physiological parameter, the signal including a visual signal or an audible signal.
12. The mechanical chest compression device of one of clauses 4 to 11, wherein the processor is further configured to: in response to removing the artifact from the physiological parameter, determining, by analyzing the physiological parameter, that blood is spontaneously circulating in the subject; and in response to determining that the blood is spontaneously circulating in the subject, causing the compressor to cease the chest compressions.
13. The mechanical chest compression device of one of clauses 4 to 12, the defibrillator being a first defibrillator, the electrical shock being a first electrical shock, wherein the processor is further configured to: output, to a second defibrillator, an instruction to administer a second electrical shock to the heart of the subject, the second shock having a different vector than the first shock.
14. The mechanical chest compression device of one of clauses 4 to 13, the physiological parameter being an electrocardiogram (ECG), wherein the processor is further configured to: in response to removing the artifact from the ECG, determine that the ECG is indicative of ventricular fibrillation (VF) or pulseless ventricular tachycardia (VT); and in response to determining that the ECG is indicative of VF or pulseless VT, output, to a defibrillator, an instruction to administer an electrical shock to a heart of the subject.
15. The mechanical chest compression device of clause 14, the defibrillator being a first defibrillator, the electrical shock being a first electrical shock, wherein the processor is further configured to: in response to determining that the ECG is indicative of VF or pulseless VT, output, to a second defibrillator, an instruction to administer a second electrical shock to the heart of the subject, the second shock having a different vector than the first shock.
16. The mechanical chest compression device of clause 15, wherein a time period between a leading edge of the first shock and a leading edge of the second shock is in a range of 1 millisecond (ms) to 200 ms.
17. The mechanical chest compression device of clause 15 or 16, wherein the second shock overlaps the first shock in time.
18. The mechanical chest compression device of one of clauses 15 to 17, wherein determining that the ECG is indicative of VF or pulseless VT includes: determining that an amplitude of the ECG has decreased greater than a threshold amount over a time period.
19. The mechanical chest compression device of one of clauses 15 to 18, wherein the processor is further configured to: determine that the first defibrillator has previously output greater than a threshold number of electrical shocks to the subject.
20. The mechanical chest compression device of one of clauses 4 to 19, further including: a cot configured to support the subject.
21. A method, including: receiving, from an external device, a first signal indicating a physiological parameter of the subject detected during a time interval in which a treatment is administered to a subject; removing, from the physiological parameter, an artifact associated with the treatment; and in response to removing the artifact from the physiological parameter, outputting a second signal indicating the physiological parameter.
22. The method of clause 21, wherein the treatment includes chest compressions, assisted ventilation, or pace pulses.
23. The method of clause 21 or 22, wherein the external device includes a monitor-defibrillator.
24. The method of clause 21, wherein the physiological parameter includes an electrocardiogram (ECG), a capnograph, a blood pressure, an airway flow, a blood flow, or a plethysmograph.
25. The method of one of clauses 21 to 24, wherein the treatment is administered at a frequency, and wherein removing the artifact associated with the treatment includes: generating a comb filter including a first band overlapping the frequency and second bands overlapping harmonics of the frequency; and applying the comb filter to the physiological parameter.
26. The method of one of clauses 21 to 25, the treatment being a first treatment, further including: in response to removing the artifact from the physiological parameter, determining, by analyzing the physiological parameter, that the subject has a treatable condition; and in response to determining that the subject has the treatable condition, generate an instruction to administer a second treatment and/or continue the first treatment.
27. The method of one of clauses 21 to 26, further including: in response to removing the artifact from the physiological parameter, determining, by analyzing the physiological parameter, that the subject has a pulse or is spontaneously breathing; and in response to determining that the subject has the pulse or is breathing, discontinuing the treatment.
28. The method of one of clauses 21 to 27, physiological parameter being a first physiological parameter, wherein determining that the subject has the pulse or is spontaneously breathing includes analyzing a second physiological parameter of the subject.
29. A system, including: a mobile device; a mechanical chest compression device including: a compressor configured to apply chest compressions to a subject; a transceiver configured to receive a first signal indicating an electrocardiogram (ECG) of the subject; and a processor configured to: identify a frequency at which the compressor applies the chest compressions to the subject; generate a comb filter including a first band overlapping the frequency at second bands overlapping harmonics of the frequency; remove a chest compression artifact from the ECG by applying the comb filter to the ECG; and in response to removing the chest compression artifact from the ECG, cause the transceiver to transmit, to the mobile device, a second signal indicating the ECG of the subject omitting the chest compression artifact.
30. The system of clause 29, wherein the first signal further indicates the physiological parameter, and wherein the processor is further configured to: in response to removing the chest compression artifact from the ECG, determine that the ECG includes a QRS complex; determine that the physiological parameter is indicative of blood flowing through a blood vessel of the subject, and in response to determining that the ECG includes a QRS complex and determining that the physiological parameter is indicative of blood flowing through the blood vessel of the subject, cause the compressor to cease the chest compressions.
31. The system of clause 29 or 30 wherein the mobile device is further configured to: receive the second signal indicating the ECG of the subject with the chest compression artifact removed; and display the ECG of the subject omitting the chest compression artifact.
32. A monitor, including: a sensor configured to detect a physiological parameter including an artifact; a transceiver configured to transmit, to a treatment device, a first signal indicating the physiological parameter including the artifact and to receive, from the treatment device, a second signal indicating the physiological parameter without the artifact; and a display configured to visually present the physiological parameter without the artifact.
33. The monitor of clause 32, wherein the monitor includes a monitor-defibrillator, and wherein the treatment device includes a mechanical chest compression device or a ventilation device.
34. The monitor of clause 32 or 33, wherein the physiological parameter includes an electrocardiogram (ECG), a capnograph, a blood pressure, an airway flow, a blood flow, or a plethysmograph.
35. The monitor of one of clauses 32 to 34, wherein the artifact includes a chest compression artifact, a ventilation artifact, or a motion artifact.
36. The monitor of one of clauses 32 to 35, wherein the artifact is associated with a treatment administered by the treatment device.
37. The monitor of one of clauses 32 to 36, further including: a processor configured to: determine, by analyzing the physiological parameter omitting the artifact, that a subject has a treatable condition; and in response to determining that the subject has the treatable condition, output a recommendation to administer a treatment to the subject.
38. The monitor of clause 37, wherein the treatable condition includes ventricular fibrillation, bradycardia, or pulseless tachycardia.
39. The monitor of clause 37 or 38, further including: a treatment circuit configured to administer the treatment to the subject.
40. The monitor of clause 39, wherein the treatment includes pace pulses or an electrical shock.
41. A method, including: detecting a physiological parameter including an artifact; transmitting, to a treatment device, a first signal indicating the physiological parameter including the artifact; receiving, from the treatment device, a second signal indicating the physiological parameter omitting the artifact; and outputting, to a user, the physiological parameter omitting the artifact.
42. The method of clause 41, wherein the physiological parameter includes electrocardiogram (ECG), a capnograph, a blood pressure, an airway flow, a blood flow, or a plethysmograph.
43. The method of clause 41 or 42, wherein the artifact includes a chest compression artifact, a ventilation artifact, or a motion artifact.
44. The method of one of clauses 41 to 43, wherein the artifact is associated with a treatment administered by the treatment device.
45. The method of one of clauses 41 to 44, further including: determining, by analyzing the physiological parameter omitting the artifact, that a subject has a treatable condition; and outputting an instruction to administer a treatment to the subject.
46. The method of clause 45, further including: receiving an input signal; and in response to receiving the input signal, generate an instruction to administer the treatment to the subject.
47. The method of clause 45 or 46, wherein the treatment includes pace pulses, an electrical shock, assisted ventilation, or chest compressions.
48. The method of one of clauses 41 to 47, further including: determining, by analyzing the physiological parameter without the artifact, that a subject has a pulse or is breathing spontaneously; and in response to determining that the subject has the pulse or is breathing spontaneously, transmitting, to the treatment device, an instruction to discontinue a treatment to the subject.
49. A method, comprising: receiving, from an external device, a first signal indicating an acceleration of the subject detected during a time interval in which a treatment is administered to a subject; removing, from the acceleration, an artifact associated with the treatment; and in response to removing the artifact from the acceleration, determining whether the subject is moving during the time interval by analyzing the acceleration.
50. The method of clause 49, wherein the treatment includes pace pulses, an electrical shock, assisted ventilation, or chest compressions.
51. The method of clause 49 or 50, wherein the acceleration of the subject is detected by a motion sensor in physical contact with the subject.
52. The method of any of clauses 49 to 51, wherein removing, from the acceleration the artifact associated with the treatment includes: identifying a frequency of the treatment; generating a comb filter including a first band overlapping the frequency and second bands overlapping harmonics of the frequency; and applying the comb filter to the acceleration.
53. The method of any of clauses 49 to 52, wherein determining whether the subject is moving includes comparing the acceleration to a threshold.
54. The method of any of clauses 49 to 53, wherein determining whether the subject is moving includes determining that the subject is spontaneously moving or is being transported during the time interval.
55. The method of any of clauses 49 to 54, wherein determining whether the subject is moving includes determining that the subject is moving during the time interval.
56. The method of clause 55, further including: in response to determining that the subject is moving, generating an instruction to cease administration of the treatment to the subject.
57. The method of clause 55 or 56, further including: in response to determining that the subject is moving, disabling a filter of a physiological parameter of the subject.
58. The method of clause 57, wherein the filter includes an adaptive filter.59. The method of any of clauses 49 to 58, the filter being a first filter, wherein determining whether the subject is moving includes determining that the subject is substantially still during the time interval, the method further including: detecting a physiological parameter of the subject during the time interval; and in response to determining that the subject is substantially still during the time interval, applying the first filter or a second filter to the physiological parameter.
60. The method of clause 59, further including: determining a condition of the subject by analyzing the physiological parameter; and ceasing or continuing the treatment based on the condition.
61. The method of clause 59 or 60, further including: determining a condition of the subject by analyzing the physiological parameter; generating a recommendation corresponding to the treatment or an additional treatment based on the condition; and outputting the recommendation.
62. A mechanical chest compression device configured to perform the method of any of clauses 49 to 61.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be used for realizing implementations of the disclosure in diverse forms thereof.

As will be understood by one of ordinary skill in the art, each implementation disclosed herein can comprise, consist essentially of or consist of its particular stated element, step, or component. Thus, the terms "include" or "including" should be interpreted to recite: "comprise, consist of, or consist essentially of." The transition term "comprise" or "comprises" means has, but is not limited to, and allows for the inclusion of unspecified elements, steps, ingredients, or components, even in major amounts. The transitional phrase "consisting of" excludes any element, step, ingredient or component not specified. The transition phrase "consisting essentially of" limits the scope of the implementation to the specified elements, steps, ingredients or components and to those that do not materially affect the implementation. As used herein, the term "based on" is equivalent to "based at least partly on," unless otherwise specified.

Unless otherwise indicated, all numbers expressing quantities, properties, conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques. When further clarity is required, the term "about" has the meaning reasonably ascribed to it by a person skilled in the art when used in conjunction with a stated numerical value or range, i.e. denoting somewhat more or somewhat less than the stated value or range, to within a range of ±20% of the stated value; ±19% of the stated value; ±18% of the stated value; ±17% of the stated value; ±16% of the stated value; ±15% of the stated value; ±14% of the stated value; ±13% of the stated value; ±12% of the stated value; ±11% of the stated value; ±10% of the stated value; ±9% of the stated value; ±8% of the stated value; ±7% of the stated value; t6% of the stated value; ±5% of the stated value; t4% of the stated value; t3% of the stated value; ±2% of the stated value; or ±1 % of the stated value.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing implementations (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate implementations of the disclosure and does not pose a limitation on the scope of the disclosure. No language in the specification should be construed as indicating any non-claimed element essential to the practice of implementations of the disclosure.

Groupings of alternative elements or implementations disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain implementations are described herein, including the best mode known to the inventors for carrying out implementations of the disclosure. Of course, variations on these described implementations will become apparent to those of ordinary skill in the art upon reading the foregoing description. The inventors expect skilled artisans to employ such variations as appropriate, and the inventors intend for implementations to be practiced otherwise than specifically described herein. Accordingly, the scope of this disclosure includes all modifications and equivalents of the subject matter recited in the claims appended hereto as permitted by applicable law. Moreover, any combination of the above-described elements in all possible variations thereof is encompassed by implementations of the disclosure unless otherwise indicated herein or otherwise clearly contradicted by context.

## Claims

1. A mechanical chest compression device, comprising:
a compressor configured to apply chest compressions to a subject; and
a processor configured to:
identify a physiological parameter of the subject;
remove, from the physiological parameter, an artifact associated with the chest compressions; and
in response to removing the artifact from the physiological parameter, outputting the physiological parameter.

2. The mechanical chest compression device of claim 1, further comprising:
a sensor configured to detect the physiological parameter.

3. The mechanical chest compression device of claim 1 or 2, wherein the physiological parameter comprises an electrocardiogram (ECG), a capnograph, a blood pressure, an airway flow, a blood flow, or a plethysmograph.

4. The mechanical chest compression device of any of claims 1-3, wherein the processor is configured to remove the artifact associated with the chest compressions by:
identifying a frequency of the chest compressions;
generating a comb filter comprising a first band overlapping the frequency and second bands overlapping harmonics of the frequency; and
applying the comb filter to the physiological parameter.

5. The mechanical chest compression device of any of claims 1-4, further comprising:
an output device,
wherein the processor is configured to output the physiological parameter by causing the output device to output a signal indicating the physiological parameter, the signal comprising a visual signal and/or an audible signal.

6. The mechanical chest compression device of any of claims 1-5, wherein the processor is further configured to:
in response to removing the artifact from the physiological parameter, determining, by analyzing the physiological parameter, that blood is spontaneously circulating in the subject; and
in response to determining that the blood is spontaneously circulating in the subject, causing the compressor to cease the chest compressions.

7. The mechanical chest compression device of any of claims 1-6, the physiological parameter being an electrocardiogram (ECG), wherein the processor is further configured to:
in response to removing the artifact from the ECG, determine that the ECG is indicative of ventricular fibrillation (VF) or pulseless ventricular tachycardia (VT); and
in response to determining that the ECG is indicative of VF or pulseless VT, output, to a defibrillator, an instruction to administer an electrical shock to a heart of the subject.

8. The mechanical chest compression device of claim 7, the defibrillator being a first defibrillator, the electrical shock being a first electrical shock, wherein the processor is further configured to:
in response to determining that the ECG is indicative of VF or pulseless VT, output, to a second defibrillator, an instruction to administer a second electrical shock to the heart of the subject, the second shock having a different vector than the first shock.

9. The mechanical chest compression device of claim 8, wherein the second shock overlaps the first shock in time, and/or
wherein determining that the ECG is indicative of VF or pulseless VT comprises:
determining that an amplitude of the ECG has decreased greater than a threshold amount over a time period.

10. The mechanical chest compression device of claim 8 or 9, wherein the processor is further configured to:
determine that the first defibrillator has previously output greater than a threshold number of electrical shocks to the subject.

11. A system, comprising:
a monitor configured to:
generate an electrocardiogram (ECG) by detecting an electrical signal indicative of cardiac activity of a subject; and
to transmit a first signal indicating the ECG of the subject; and
a mechanical chest compression device according to claim 1, wherein the processor is configured to:
identify a frequency at which the compressor applies the chest compressions to the subject;
generate a comb filter comprising a first band overlapping the frequency and second bands overlapping harmonics of the frequency;
remove a chest compression artifact from the ECG by applying the comb filter to the ECG; and
in response to removing the chest compression artifact from the ECG, cause the transceiver to transmit a second signal indicating the ECG of the subject omitting the chest compression artifact.

12. The system of claim 11, wherein the monitor is further configured to detect a physiological parameter of the subject and to transmit a third signal indicating the physiological parameter,
wherein the transceiver is further configured to receive the third signal indicating the physiological parameter, and
wherein the processor is further configured to:
in response to removing the chest compression artifact from the ECG, determine that the ECG comprises a QRS complex;
determine that the physiological parameter is indicative of blood flowing through a blood vessel of the subject, and
in response to determining that the ECG comprises a QRS complex and determining that the physiological parameter is indicative of blood flowing through the blood vessel of the subject, cause the compressor to cease the chest compressions.

13. The system of claim 11 or 12, wherein the monitor is further configured to:
receive the second signal indicating the ECG of the subject omitting the chest compression artifact; and
display the ECG of the subject omitting the chest compression artifact.

14. A method, comprising:
receiving, from an external device, a first signal indicating a physiological parameter of the subject detected during a time interval in which a treatment is administered to a subject;
removing, from the physiological parameter, an artifact associated with the treatment; and
in response to removing the artifact from the physiological parameter, outputting a second signal indicating the physiological parameter.

15. The method of claim 14, wherein removing the artifact associated with the treatment comprises:
generating a comb filter comprising a first band overlapping a frequency of administration of the treatment and second bands overlapping harmonics of the frequency; and
applying the comb filter to the physiological parameter.
